# EUROPEAN PATENT SPECIFICATION

(11) **EP 2 457 582 B1**
(45) Date of publication and mention of the grant of the patent: **30.04.2014**
(21) Application number: 12001094.7
(22) Date of filing: 16.07.2004
(51) Int. Cl.: A61K 38/55, A61K 38/04, A61K 31/444, A61K 31/4015, A61P 33/14

(54) **Method and compositions for controlling ectoparasites**
Verfahren und Zusammensetzungen zur Kontrolle von Ektoparasiten
Procédé et compositions pour le contrôle d'ectoparasites

(30) Priority: 16.07.2003 US 487717 P; 16.07.2003 AU 2003903686
(43) Date of publication of application: 30.05.2012
(62) Divisional of application: 04737575.3
(73) Proprietor: Hatchtech Pty Ltd, Parkville, VIC 3052 (AU)
(72) Inventor: Bowles, Vernon Morrison, Glen Iris 3146 Victoria (AU)
(74) Representative: Greenwood, Matthew David

(56) References cited:
- WO-A1-95/35031
- CA-A1- 2 394 002
- CA-A1- 2 408 209
- YOUNG ANNA R ET AL: "Characterisation of proteases involved in egg hatching of the sheep blowfly, Lucilia cuprina", INTERNATIONAL JOURNAL FOR PARASITOLOGY, vol. 30, no. 8, 2000, pages 925-932, XP002674149,
- ROGERS W P ET AL: "The mechanism of hatching of eggs of Haemonchus contortus", INTERNATIONAL JOURNAL OF PARASITOLOGY, vol. 7, no. 1, 1977, pages 61-65, XP023651980, [retrieved on 1977-02-01]
- DATABASE BIOSIS [Online] BIOSCIENCES INFORMATION SERVICE, PHILADELPHIA, PA, US; April 1998 (1998-04), DAY T A ET AL: "The metalloprotease, inhibitor, 1,10-phenanthroline affects Schistosoma mansoni motor activity, egg laying and viability", XP002674150, Database accession no. PREV199800275411 & DAY T A ET AL: PARASITOLOGY, vol. 116, no. 4, April 1998 (1998-04), pages 319-325,
- VIEIRA C ET AL: "Pro- and anti-inflammatory actions of ricinoleic acid: similarities and differences with capsaicin.", NAUNYN-SCHMIEDEBERG'S ARCHIVES OF PHARMACOLOGY, vol. 364, no. 2, 2001, pages 87-95, XP002548583,
- DATABASE BIOSIS [Online] BIOSCIENCES INFORMATION SERVICE, PHILADELPHIA, PA, US; 1996, SUZUKI HIDEYUKI et al: "Neutral endopeptidase modulates VIP-induced vasodilation in hamster cheek pouch vessels in situ", XP002548582, Database accession no. PREV199699196396
- REED B J ET AL: "Aminopeptidases as potential targets for the control of the Australian sheep blowfly, Lucilia cuprina", INTERNATIONAL JOURNAL FOR PARASITOLOGY, vol. 29, no. 6, 1999, pages 839-850, XP002548584,
- DATABASE WPI Week 200342 Thomson Scientific, London, GB; AN 2003-443682 XP002548581, & JP 2002 363008 A (JUKA LIFETEK KK) 18 December 2002 (2002-12-18)

## Description

### BACKGROUND OF THE INVENTION

### FIELD OF THE INVENTION

The present invention relates to methods and compositions for controlling ectoparasites. In particular, the invention relates to methods and compositions for inhibiting hatching of an ectoparasite egg. The invention also provides methods and compositions for preventing or treating ectoparasite infestation. The invention also relates to methods for identifying compounds that can inhibit ectoparasite egg hatching.

### DESCRIPTION OF THE PRIOR ART

Ectoparasites including some insects cause significant pest problems in a wide variety of animals and plants. In particular, ectoparasites typically can annoy, bite, and cause infections to humans and domesticated animals. Of particular concern is the presence and effect of such parasites on humans, household pets or companion animals, such as dogs and cats, and other domesticated animals, such as horses.

Various compositions and application techniques are known for controlling or eliminating biting or blood-sucking pests (ectoparasites), such as fleas, ticks, flies, lice and mites. Over the years a host of aerosols and space sprays, liquids, soaps, shampoos, wettable powders, granules, baits, and dusts, have been proposed for the control of such ectoparasites.

Conventional control measures for ectoparasites have relied on the use of chemical insecticides, for example chlorinated hydrocarbons (DDT, endosulfan etc), and synthetic pyrethroids (cypermethrin, deltamethrin). Problems associated with the use of chemical pesticides include the development of resistance by target ectoparasites, the persistence of the chemicals in the environment and in plant and animal tissues, and the harmful effects on host and non-target organisms.

Other types of ectoparasiticides include insecticides, such as insect growth regulators (IGRs) that are known to interfere with chitin synthesis and insecticidal bacterial toxins (eg. Bacillus thuringiensis (Bt) toxins). More useful groups of insecticides are those having high insecticidal activity and low environmental persistence, such as organophosphates and synthetic pyrethroids. However, a significant problem associated with these insecticides is the development of resistance by target insects.

For example, insecticidal agents used to treat lice are described in EP 0191236 and U.S. Pat. No. 5,288,483. A significant disadvantage of using these agents is that lice can become resistant. The need for further treatment increases the exposure to these harsh agents and increases the cost. Additionally, clinicians and parents are reluctant to treat children with agents that can also prove toxic to human beings. Moreover, many of these compounds have unpleasant odors or other undesirable properties, causing noncompliance by the patient, leading to re-infestation of the individual, and spreading of the infestation to others. In addition, the harshness of these agents makes them unsuitable for use as prophylactics.

In the case of head lice infestation, home remedies such as application of corn oil, olive oil, eucalyptus oil, neem oil, coconut oil, mayonnaise, or petroleum jelly for a period of time sufficient to kill the lice (e.g. overnight) are not practical or completely effective. A further disadvantage of methods to treat head lice is the requirement of removing the nits from the hair in a separate treatment step. The removal of nits has typically been done by hand using special fine-tooth combs. Use of combing alone to treat head lice has the disadvantage that the lice can hold onto the hair shafts using their claws or escape by crawling away from the area being combed. This labor intensive method requires daily combing, is painful, and is unpleasant since the lice are active, visible and crawling.

There is a significant need for improved control of lice throughout the world. In particular, there are well-documented failures of products aimed at treating lice. The development of resistance of lice to many of the currently used chemicals including permethrin, pyrethrin and malathion is considered a major factor in treatment failures. In addition, inappropriate formulations containing suboptimal actives are also believed to be in part responsible for resistance development. More recently there has been significant growth in the market for herbal products for treating head lice however there is very little published evidence from properly conducted trials to enable an effective assessment of these products to be made. Furthermore while a number of products claim to possess ovicidal activity the evidence for this in the field is far from convincing hence it is common for products to recommend that following an initial treatment a second treatment should be given between 7-14 days later to kill newly emerged nymphs.

Recently attention has focused on insect proteases that may provide a possible means of ectoparasite control. Proteases perform a variety of functions in the organism including the regulation and breakdown of proteins and peptides, and thus assist with digestion. They are also involved in tissue reorganization during embryo development, molting and pupation. Proteases are a widely variable group of enzymes and include digestive proteases that vary considerably both in number and in catalytic properties within and between species. For example, trypsin-like serine proteases have been recognized to be involved in the key growth regulatory area of molting (Samuels R.I. and Paterson C.J., Comparative Biochemistry and Physiology, 1995, 110B: 661-669).

Protease inhibitors have been suggested to be a useful alternative to the chemical control methods, particularly where the ectoparasites have become resistant to these chemical pesticides. In particular, serine and cysteine protease inhibitors have been shown to reduce the larval growth and/or survival of various insects (Dymock et. al., New Zealand Journal of Zoology, 1992, 19: 123-131). Growth inhibition has been achieved with inhibitors of principal digestive enzymes of the gut and have been targeted at ectoparasite larvae or mature parasites. However, little is known about other types of activity and function of various classes of protease inhibitors. A common problem of existing ectoparasiticides is that they do not affect the ectoparasite eggs and therefore application of the parasiticides to hosts often require repeated treatment or prolonged exposure to the parasiticide for it to be effective. This is not only inconvenient but also increases risks to the environment and to the host.

Young, et al. (2000) Int J Parasit, 30:925-932 relates to the characterisation of proteases involved in egg hatching of the sheep blowfly, *Lucilia cuprina*. The studies indicate a role for serine and/or metallo-proteases in facilitating *L. cuprina* egg hatch.

There remains a need for providing alternative methods and compositions that are effective in inhibiting ectoparasite egg hatching to provide efficient control of etoparasites.

According to an aspect of the invention there is provided use of at least one metal chelating agent or a pharmaceutically, veterinary or agriculturally acceptable salt thereof, for the manufacture of a topical composition for use in inhibiting hatching of an ectoparasite egg on a human or animal host, wherein the at least one metal chelating agent is a compound of formula (Ia): 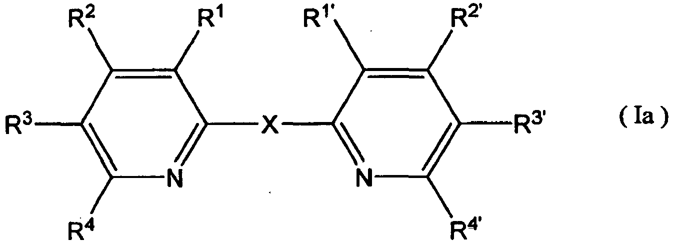
wherein X is a covalent bond, CH₂-Z-CH₂ or -Z-
wherein R¹ and R^{1'} are independently selected from hydrogen, or C₁₋₃alkyl
wherein at least one of R², R^{2'}, R³, R^{3'}, R⁴ and R^{4'} is a methyl group and the remaining R², R^{2'}, R³, R^{3'}, R⁴ and R^{4'} are independently selected from hydrogen or C₁₋₃ alkyl; and Z is selected from a covalent bond, -NH-, -O-, -S-, -C(O)- and -C(S)-.

A further aspect provides a method for inhibiting hatching of an ectoparasite egg, excluding use on a human or animal host, comprising exposing the ectoparasite egg to a topical composition comprising at least one metal chelating agent, wherein the at least one metal chelating agent is a compound of formula (Ia): 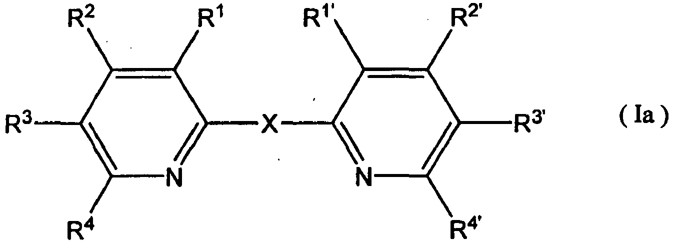
wherein X is a covalent bond, CH₂-Z-CH₂ or -Z-
wherein R¹ and R^{1'} are independently selected from hydrogen, or C₁₋₃alkyl
wherein at least one of R², R^{2'}, R³, R^{3'}, R⁴ and R^{4'} is a methyl group and the remaining R², R^{2'}, R³, R^{3'}, R⁴ and R^{4'} are independently selected from hydrogen or C₁₋₃ alkyl; and Z is selected from a covalent bond, -NH-, -O-, -S-, -C(O)- and -C(S)-;
or a pharmaceutically, veterinary or agriculturally acceptable salt thereof.

A further aspect provides use of an effective amount of at least one metal chelating agent or a pharmaceutically, veterinary or agriculturally acceptable salt thereof, for the manufacture of a topical medicament for use in treating ectoparasite infestation in a human or animal host, wherein the at least one metal chelating agent is a compound of formula (Ia): 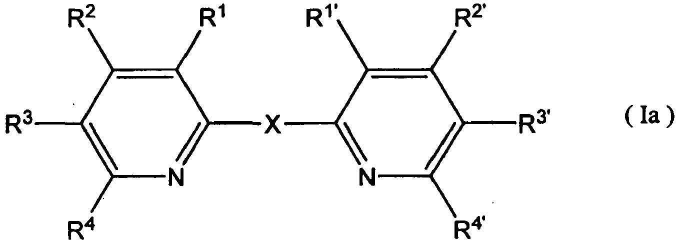
wherein X is a covalent bond, CH₂-Z-CH₂ or -Z-
wherein R¹ and R^{1'} are independently selected from hydrogen, or C₁₋₃alkyl
wherein at least one of R², R^{2'}, R³, R^{3'}, R⁴ and R^{4'} is a methyl group and the remaining R², R^{2'}, R³, R^{3'}, R⁴ and R^{4'} are independently selected from hydrogen or C₁₋₃ alkyl; and-Z is selected from a covalent bond, -NH-, -O-, -S-, -C(O)- and -C(S)-.

A further aspect provides a method of treating ectoparasite infestation, excluding use on a human or animal host, comprising exposing the host to an effective amount of at least one metal chelating agent, wherein the at least one metal chelating agent is a compound of formula (Ia): 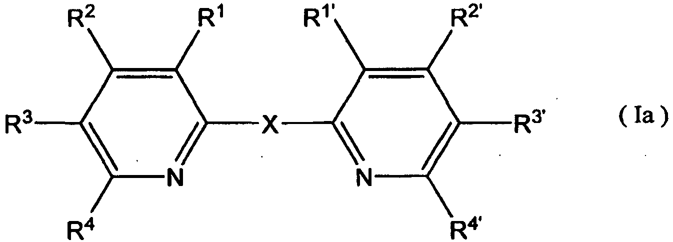
wherein X is a covalent bond, CH₂-Z-CH₂ or -Z-
wherein R¹ and R^{1'} are independently selected from hydrogen, or C₁₋₃alkyl
wherein at least one of R², R^{2'}, R³, R^{3'}, R⁴ and R^{4'} is a methyl group and the remaining R², R^{2'}, R³, R^{3'}, R⁴ and R^{4'} are independently selected from hydrogen or C₁₋₃ alkyl; and-Z is selected from a covalent bond, -NH-, -O-, -S-, -C(O)- and -C(S)-;
or a pharmaceutically, veterinary or agriculturally acceptable salt thereof.

The ectoparasite egg may be present on: host plants including cereal crops, fruit trees, cotton, oil seed crops, ornamental plants, flowers, vine crops, root crops, pasture plants and vegetables, or on breeding sites such as: houses and buildings, enclosures for domestic and farming animals, carpets, blankets, curtains and furniture.

Z may be -NH-, -O- or S.

The compound of formula (Ia) may be selected from: 6,6'-dimethyl-2,2'-dipyridyl, 5,5'-dimethyl-2,2'-dipyridyl, 4,4'-dimethyl-2,2'-dipyridyl, or a pharmaceutically, veterinary or agriculturally acceptable salt thereof.

The ectoparasite egg may be laid by an ectoparasite of a species from an order selected from the group consisting of Lepidoptera, Hemiptera, Orthoptera, Psocoptera, Hymenoptera, Isoptera, Coleoptera, Dictyoptera, Thysanoptera, Homoptera, Diptera, Anaplura, Malophaga, Siphoneptera, Arachnida and Phthiraptera.

The ectoparasite egg may be laid by an ectoparasite of a species selected from the group consisting of Helicoverpa spp. Crocidolomia pavonana (Cabbage cluster caterpillar), Pieris rapae (Cabbage white butterfly), Phthorimaea operculella (Potatoe moth), Chrsyodexis spp. (Tobacco loopers), Plutella xylostella (Diamondback moth), Eiphyas postvittana (Walker)(light briown apple moth), Bovicola ovis (Sheep louse), Bovicola bovis, Haemotopinus eurysternus (short-nosed cattle louse), Linognathus vituli (long nosed cattle louse), Solenoptes scabiei suis, Sarcoptes scabiei bovis, Psoroptes ovis, Pthirus pubis, Pediculus humanus capitus, Pedicululs humanus humanus, Sarcoptes scabiei var, humani and Dermatophgoides spp.

The ectoparasite infestation may be a lice infestation.

The metal chelating agent may be applied simultaneously, separately or sequentially with a second ectoparasiticide. The second ectoparasiticide may control nymphs and/or adult ectoparasites.

The composition may be formulated for use in a direct topical application in a form selected from the group consisting of a dip, spray, aerosol, shampoo, mousse, emulsion, solution cream, dust, lotion, foams, microemulsions and jellies.

The ectoparasite egg may be human head lice eggs and the composition is formulated for use in a topical application to human scalp and is in a form selected from the group consisting of a dip, spray, aerosol, shampoo, mousse, emulsion, solution cream, dust, lotion, foams, microemulsions and jellies.

The composition may include: water, salt solutions, alcohols, polyethylene glycols, gelatine, lactose, magnesium sterate, or silicic acid.

A further aspect provides at least one metal chelating agent or a pharmaceutically, veterinary or agriculturally acceptable salt thereof, for use in inhibiting hatching of an ectoparasite egg on a human or animal host, wherein the at least one metal chelating agent is a compound of formula (Ia): 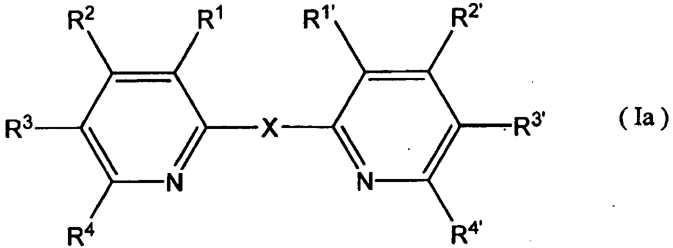
wherein X is a covalent bond, CH₂-Z-CH₂ or -Z-
wherein R¹ and R^{1'} are independently selected from hydrogen, or C₁₋₃alkyl
wherein at least one of R², R^{2'}, R³, R^{3'}, R⁴ and R^{4'} is a methyl group and the remaining R², R^{2'}, R³, R^{3'} , R⁴ and R^{4'} are independently selected from hydrogen or C₁₋₃ alkyl; and Z is selected from a covalent bond, -NH-, -O-, -S-, -C(O)- and -C(S)-.

A further aspect provides method of inhibiting hatching of an ectoparasite egg, excluding use on a human or animal host, comprising exposing the ectoparasite egg to at least one metal chelating agent or a pharmaceutically, veterinary or agriculturally acceptable salt thereof, wherein the at least one metal chelating agent is a compound of formula (Ia): 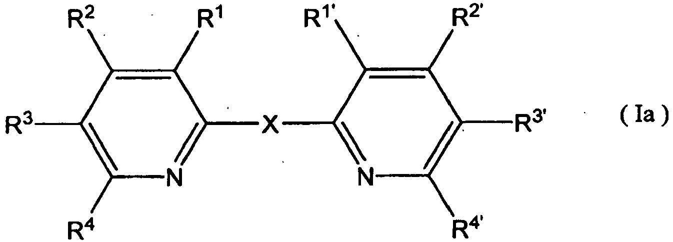
wherein X is a covalent bond, CH₂-Z-CH₂ or -Z-
wherein R¹ and R^{1'} are independently selected from hydrogen, or C₁₋₃alkyl
wherein at least one of R², R^{2'}, R³, R^{3'}, R⁴ and R^{4'} is a methyl group and the remaining R², R^{2'}, R³, R^{3'}, R⁴ and R^{4'} are independently selected from hydrogen or C₁₋₃ alkyl; and Z is selected from a covalent bond, -NH-, -O-, -S-, -C(O)- and -C(S)-.

The ectoparasite egg may be present on: host plants including cereal crops, fruit trees, cotton, oil seed crops, ornamental plants, flowers, vine crops, root crops, pasture plants and vegetables, or on breeding sites such as: houses and buildings, enclosures for domestic and farming animals, carpets, blankets, curtains and furniture.

The compound of formula (Ia) may be 5,5' dimethyl-2-2'dipyridyl, or a pharmaceutically, veterinary or agriculturally acceptable salt thereof.

### BRIEF DESCRIPTION OF THE FIGURES

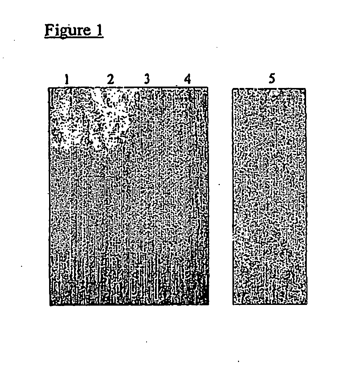

**Figure 1****:** shows a gelatine substrate SDS-PAGE analysis of protease activity of washings obtained from various samples of hair and lice eggs following staining of the gel with Coomassie blue and destaining. Lane 1 shows protease activity detected in the washings obtained from unhatched lice eggs within 12 hours of hatching. Protease activity was in the higher molecular weight region of the SDS gel. Lane 2 shows protease activity detected in the washings from hair indicating the presence of a number of highly active and stable proteases likely to be of maternal origin. Lane 3 shows a hair sample that was washed with a 1% solution of sodium hypochlorite for 1 minute followed by a number of water washes in an attempt to remove these contaminating proteases. This treatment was able to remove the maternal proteases resulting in no protease species being detected in the hair only sample. Lane 4 shows protease activity detected in the washings from eggs within 12 hours of egg hatching treated with sodium hypochlorite (as described above). This treatment removed the protease activity that was observed in the unwashed sample (compare to lane 1). Lane 5 shows the presence of one or two high molecular weight protease species in egg washings from lice eggs that had been pretreated with sodium hypochlorite and allowed to hatch. These proteases were specifically associated with the lice eggs at the time of egg hatching.

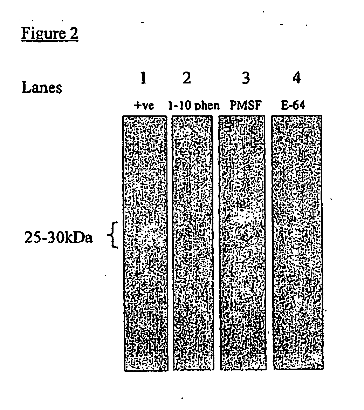

**Figure 2****:** shows a Coomassie stain of inhibitor treated gelatine SDS-PAGE gels of the egg shell washings from lice eggs following hypochlorite treatment. Three bands were evident at approximately 25-30 kDa (bracketed). Lane 1, ESW positive control no inhibitor treatment, lane 2, ESW after treatment with 10mM 1,10-phenanthroline, lane 3, ESW after treatment with 5 mM PMSF and lane 4 ESW after treatment with 10µm E-64. Incubation was performed at 37°C for 3 hours. Note the significant reduction in protease activity following treatment with 1,10-phenanthroline (lane 2 bracketed region). No reduction in protease activity of the ESW was observed when the aspartic inhibitor pepstatin was used (data not shown).

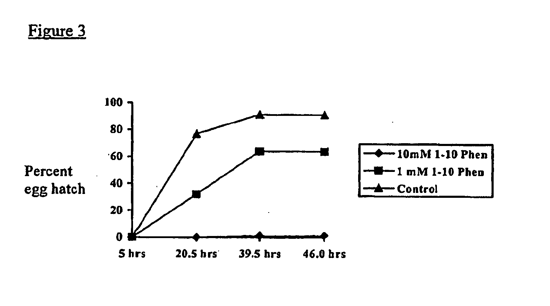

**Figure 3****:** shows the effect of 1,10-phenanthroline on egg hatching in lice. Eggs were treated 5 days post laying and then hatching observed over time.

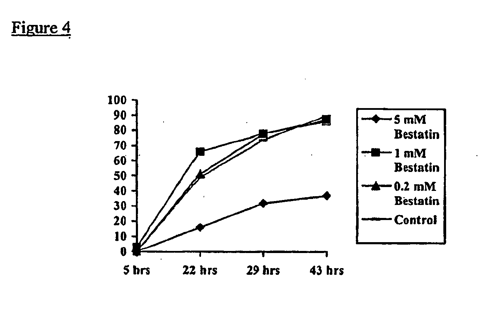

**Figure 4****:** shows the effect of Bestatin on egg hatching in lice. Eggs were treated 5 days post laying and then hatching observed over time.

### DETAILED DESCRIPTION

As used herein, the term "metal chelating agent" refers to a compound comprising at least two heteroatoms able to simultaneously coordinate with a metal ion, at least one of the two heteroatoms being selected from nitrogen, sulfur, oxygen or phosphorus, wherein the compound comprises at least one carbocyclic ring substituted with at least one heteroatom and/or a substituent containing at least one heteroatom, or the compound comprises at least one heterocyclic ring containing at least one heteroatom, and wherein said heterocyclic ring is optionally substituted with at least one heteroatom and/or a substituent containing at least one heteroatom. Preferably the metal chelating agent contains an aryl or heteroaryl ring. More preferably, the metal chelating agent comprises at least one nitrogen heteroatom. Preferably the metal chelating agent is non-intercalating.

As used herein, the term "metalloprotease inhibitor" refers to a molecule, compound, protein or agent that inhibits the activity of a metalloprotease associated with ectoparasite egg hatching. The inhibition may be inhibition of the expression of the metalloprotease or inhibition of the enzymatic activity of the metalloprotease. Preferred metalloprotease inhibitors are metal chelating agents.

Preferred metal chelating agents and metalloprotease inhibitors are selected from biaryl compounds, peptides and amino acid derivatives, tetracyclic antibiotics and thioureas. Preferred biaryl compounds include bipyridyl compounds and 1,10-phenanthroline compounds.

A metal chelating agent or metalloprotease inhibitor can be a compound of formula (I): 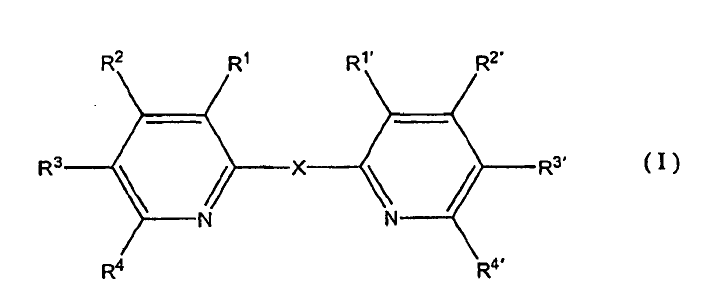
wherein X is selected from a covalent bond, -C(R⁵)₂-, -Z- or -C(R⁵)₂-Z-C(R⁵)₂-; R¹ and R^{1'} are independently selected from hydrogen, C₁₋₆alkyl, C₁₋₆alkenyl, C₂₋₆alkynyl, hydroxy, C₁₋₆alkoxy, thiol, C₁₋₆alkylthio, CO₂H, CO₂C₁₋₆alkyl, SO₃H, SO₃C₁₋₆alkyl, NH₂, NHC₁₋₆alkyl or N(C₁₋₆alkyl)₂, or R¹ and R^{1'} taken together are -C(R⁵)₂-, -C(R⁵)₂-C(R⁵)₂-, -CR⁵=CR⁵-, C(O), C(S) or NH;
R², R^{2'}, R³, R^{3'}, R⁴ and R^{4'} are independently selected from hydrogen, C₁₋₆alkyl, C₂₋₆alkenyl, C₂₋₆alkynyl, hydroxy, C₁₋₆alkoxy, thiol, C₁₋₆alkylthiol, CO₂H, CO₂C₁₋₆alkyl, SO₃H SO₃C₁₋₆alkyl, NH₂, NHC₁₋₆alkyl or N(C₁₋₆alkyl)₂, or -CH₂CHNH(CO₂H); or
R² and R³ or R³ and R⁴ and/or R^{2'} and R^{3'} or R^{3'} and R^{4'} taken together with the carbon atoms to which they are attached form a 5 or 6 membered carbocyclic or heterocyclic ring; each R⁵ is independently selected from hydrogen, C₁₋₆alkyl, C₂₋₆alkenyl, C₂₋₆alkynyl, hydroxy, C₁₋₆alkoxy, thiol, C₁₋₆alkylthiol, CO₂H, CO₂C₁₋₆alkyl, SO₃H, SO₃C₁₋₆alkyl, NH₂, NHC₁₋₆alkyl or N(C₁₋₆alkyl)₂; and
Z is selected from a covalent bond, -NH-, -O-, -S-, -C(O)- and -C(S)-;
or a pharmaceutically, veterinary or agriculturally acceptable salt thereof.

Preferred compounds of formula (I) have at least one of the following features:
R¹ and R^{1'} are independently selected from C₁₋₆alkyl, C₂₋₆alkenyl, C₁₋₆alkynyl, hydroxy, C₁₋₆alkoxy, thiol, C₁₋₆alkylthio, CO₂H, CO₂C₁₋₆alkyl, SO₃H, SO₃C₁₋₆alkyl NH₂, NHC₁₋₆ alkyl or N(C₁₋₆alkyl)₂,, more preferably hydrogen or C₁-C₃alkyl, even more preferably hydrogen or methyl;
R² and R^{2'} are independently hydrogen or C₁₋₃alkyl, more preferably hydrogen;
R³, R^{3'}, R⁴ and R^{4'} are independently selected from hydrogen, C₁₋₆alkyl, C₂₋₆alkenyl, C₂₋₆alkynyl, C₁₋₆alkoxy, C₁₋₆alkylthiol or CO₂C₁₋₆alkyl, preferably hydrogen or C₁₋₃alkyl, more preferably hydrogen or methyl;
each R⁵ is independently selected from hydrogen, C₁₋₆alkyl, C₂₋₆alkenyl, C₂₋₆alkynyl, C₁₋₆alkoxy, C₁₋₆alkylthiol or CO₂C₁₋₆alkyl, preferably hydrogen or C₁₋₃alkyl, more preferably hydrogen or methyl;
X is a covalent bond, -CH₂-Z-CH₂- or Z, preferably a covalent bond; and
Z is -NH-, -O- or -S-, preferably -NH-.

Preferred compounds of formula (I) include
2,2'-dipyridyl,
6,6'-dimethyl-2,2'-dipyridyl, and
5,5'-dimethyl-2,2'-dipyridyl,
or a pharmaceutically, veterinary or agriculturally acceptable salt thereof.

A metal chelating agent or metalloprotease inhibitor can also be a compound of formula (II): 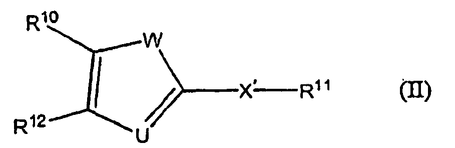
wherein X' is selected from a covalent bond, -C(R¹³)₂-, Z' or C(R¹³)₂-Z'-C(R¹³)₂-;
U is selected from N or C(R¹³);
W is selected from -NH-, -S- or -O-;
Z' is selected from a covalent bond, -NH-, -O-, -S-, -C(O)-, or -C(S)-;
R¹⁰ is selected from hydrogen, C₁₋₆alkyl, C₂₋₆alkenyl, C₂₋₆alkynyl, hydroxy, C₁₋₆alkoxy, thiol, C₁₋₆alkylthiol, CO₂H CO₂C₁₋₆alkyl, SO₃H, SO₃C₁₋₆alkyl, NH₂, NH(C₁₋₆alkyl), N(C₁₋₆alkyl)₂, or -(CH₂)ₙR¹⁴;
R¹¹ is selected from (CH₂)ₘaryl or (CH₂)ₘheteroaryl wherein each aryl or heteroaryl is optionally substituted with one or more C₁₋₆alkyl, C₂₋₆alkenyl, C₂₋₆alkynyl, hydroxy, C₁₋₆ alkoxy; thiol, C₁₋₆alkylthiol, CO₂H, CO₂C₁₋₆alkyl, SO₃H, SO₃C₁₋₆alkyl, NH₂, NH(C₁₋₆ alkyl), N(C₁₋₆alkyl)₂, or halo;
each R¹² is independently selected from hydrogen, C₁₋₆alkyl, C₂₋₆alkenyl, C₂₋₆alkynyl, hydroxy, C₁₋₆alkoxy, thiol, C₁₋₆alkylthiol, CO₂H, CO₂C₁₋₆alkyl, SO₃H, SO₃C₁₋₆alkyl, NH₂, NH(C₁₋₆alkyl), N(C₁₋₆alkyl)₂, or -(CH₂)ₙR¹⁴; or
R¹⁰ and R¹² together with the carbon atoms to which they are attached form a 5 or 6 membered carbocyclic or heterocyclic ring;
each R¹³ is independently selected from hydrogen, C₁₋₆alkyl, C₂₋₆alkenyl, C₂₋₆alkynyl, hydroxy, C₁₋₆alkoxy, thiol, C₁₋₆alkylthiol, CO₂H CO₂C₁₋₆alkyl, SO₃H, SO₃C₁₋₆alkyl, NH₂, NH(C₁₋₆alkyl), N(C₁₋₆alkyl)₂, or -(CH₂)ₙR¹⁴;
R¹⁴ is selected from NH₂, OH, SH or CO₂H;
m is 0 or an integer from 1 to 4; and
n is an integer from 1 to 4;
or a pharmaceutically, veterinary or agriculturally acceptable salt thereof.

Preferred compounds of formula (II) have at least one of the following features:
X is a covalent bond or -CH₂-Z-CH₂-;
UisN;
W is NH or S;
Z' is NH;
R¹⁰ is hydrogen, C₁₋₆alkyl, C₂₋₆alkenyl, C₂₋₆alkynyl, or (CH₂)ₙR¹⁴, preferably hydrogen, C₁₋₃alkyl or (CH₂)ₙR¹⁴;
R¹¹ is phenyl, phenyl substituted with C₁₋₃alkyl or halo, thiophene, pyridine, pyridinylmethyl, imidazole or imidazole substituted with one or two C₁₋₃alkyl;
R¹² is hydrogen, C₁₋₆alkyl, C₂₋₆alkenyl, C₂₋₆alkynyl, or (CH₂)ₙR¹⁴, preferably hydrogen, C₁₋₃alkyl or (CH₂)ₙR¹⁴; or
R¹⁰ and R¹² together with the carbon atoms to which they are attached form a fused phenyl ring;
R¹³ is hydrogen or C₁₋₃alkyl, preferably hydrogen or methyl;
R¹⁴ is NH₂ or CO₂H;
m is 0 or 1; and
nis 1 or 2.

A metal chelating agent or metalloprotease inhibitor can also be selected from a compound of formula (III): 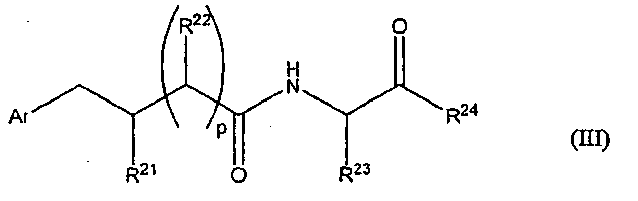
wherein Ar is phenyl, naphthyl or indolyl optionally substituted with one or more C₁₋₆alkyl, C₂₋₆alkenyl, C₂₋₆alkynyl, hydroxy, C₁₋₆alkoxy, thiol, C₁₋₆alkylthiol, CO₂H, CO₂C₁₋₆alkyl, SO₃H, SO₃C₁₋₆alkyl, NH₂, NH(C₁₋₆alkyl), N(C₁₋₆alkyl)₂;
R²¹ is selected from NH₂, NHR²⁵ or -CH₂SR²⁵;
R²² is selected from hydrogen, hydroxy or C₁₋₆alkoxy;
R²³ is selected from hydrogen, C₁₋₆alkyl, C₂₋₆alkenyl or C₂₋₆alkynyl;
R²⁴ is selected from OH, OR²⁶, NH₂, NHC₁₋₆alkyl or N(C₁₋₆alkyl)₂;
R²⁵ is selected from hydrogen, C(O)C₁₋₆alkyl wherein the alkyl is optionally substituted with -SH or -OH;
R²⁶ is selected from C₁₋₆alkyl, C₂₋₆alkenyl, C₂₋₆alkynyl or benzyl; and
p is 0 or 1,
or a pharmaceutically, veterinary or agriculturally acceptable salt thereof.

Preferred compounds of formula (III) have at least one of the following features:
Ar is phenyl or naphthyl;
R²¹ is NH₂, -NHC(O)C₁₋₆alkyl optionally substituted with SH, -CH₂SC(O)C₁₋₆alkyl or CH₂SH;
R²² is hydrogen or hydroxy;
R²³ is hydrogen or C₁₋₃alkyl, preferably hydrogen or methyl;
R²⁴ is OH, NH₂ or Obenzyl; and
p is 0 or 1.

Preferred compounds of formula III include Bestatin and Thiorophan or a pharmaceutically, veterinary or agriculturally acceptable salt thereof.

A metal chelating agent or metalloprotease inhibitor can also be a compound of formula (IV): 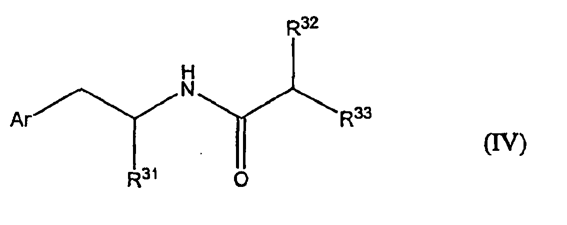
wherein Ar is phenyl, naphthyl or indolyl optionally substituted with one or more C₁₋₆alkyl, C₂₋₆alkenyl, C₂₋₆alkynyl, hydroxy, C₁₋₆alkoxy, thiol, C₁₋₆alkylthiol, CO₂H, CO₂C₁₋₆alkyl, SO₃H, SO₃C₁₋₆alkyl, NH₂, NH(C₁₋₆alkyl), N(C₁₋₆alkyl)₂;
R³¹ is selected from CO₂H, CO₂C₁₋₆alkyl, CO₂C₂₋₆alkenyl, CO₂C₂₋₆alkynyl, CONH₂, CONH(C₁₋₆alkyl) or CON(C₁₋₆alkyl)₂;
R³² is selected from hydrogen, C₁₋₆alkyl, C₂₋₆alkenyl, C₂₋₆alkynyl, hydroxy, C₁₋₆alkoxy, thiol, C₁₋₆alkylthiol, CO₂H, CO₂C₁₋₆alkyl, SO₃H, SO₃C₁₋₆alkyl, NH₂, NH(C₁₋₆alkyl), N(C₁₋₆alkyl)₂, CH₂CH₂CO₂H, CH₂CH₂CONH₂, CH₂CH₂OH CH₂CH₂SH; and
R³³ is selected from C₁₋₆alkyl, C₂₋₆alkenyl, C₂₋₆alkynyl, hydroxy, C₁₋₆alkoxy, thiol, C₁₋₆alkylthiol, CO₂H, CO₂C₁₋₆alkyl, SO₃H SO₃C₁₋₆alkyl, NH₂, NH(C₁₋₆alkyl), N(C₁₋₆alkyl)₂, CH₂CO₂H, CH₂CO₂C₁₋₆alkyl, CH₂CONH₂, CH₂OH, or CH₂SH, or a pharmaceutically, veterinary or agriculturally acceptable salt thereof.

Preferred compounds of formula (IV) have at least one of the following features:
Ar is phenyl or indolyl,
R³¹ is CO₂H or CONH₂,
R³² is C₁₋₆alkyl, CH₂CH₂CO₂H, CH₂CH₂CONH₂, CH₂CH₂OH, or CH₂CH₂SH,
R³³ is CH₂CO₂H, CH₂CONH₂, CH₂OH, or CH₂SH.

A metal chelating agent or metalloprotease inhibitor can also be a compound of formula (V): 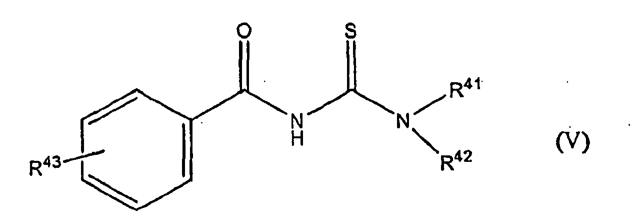
wherein R⁴¹ and R⁴² are independently selected from hydrogen, C₁₋₆alkyl, C₂₋₆alkenyl, C₂₋₆alkynyl or R⁴¹ and R⁴² taken together with the nitrogen to which they are attached form a 5 or 6 membered heterocyclic ring which is optionally substituted with one or more C₁₋₆alkyl, C₂₋₆alkenyl or C₂₋₆alkynyl groups; and
R⁴³ is selected from hydrogen, C₁₋₆alkyl, C₂₋₆alkenyl, C₂₋₆alkynyl, hydroxy, C₁₋₆alkoxy, thiol, C₁₋₆alkylthiol, CO₂H, CO₂C₁₋₆alkyl, SO₃H, SO₃C₁₋₆alkyl, NH₂, NHC₁₋₆alkyl or N(C₁₋₆alkyl)₂;
or a pharmaceutically, veterinary or agriculturally acceptable salt thereof.

Preferred compounds of formula (V) have at least one of the following features:
R⁴¹ and R⁴² are independently selected from C₁₋₆alkyl or taken together with the nitrogen to which the are attached form a piperidine, piperazine, N-methylpiperazine or morpholine group;
R⁴³ is hydrogen, C₁₋₆alkyl, C₂₋₆alkenyl or C₂₋₆alkynyl.

A metal chelating agent or metalloprotease inhibitor can also be a tetracyclic antibiotic selected from the group consisting of tetracycline, doxycycline or minocycline or a pharmaceutically, veterinary or agriculturally acceptable salt thereof.

A metal chelating agent or metalloprotease inhibitor can also be selected from 1-[(2S)-3-mercapto-2-methyl-1-oxopropyl]-L-proline (Captopril) or N-(alpha-rhamnopyranosyloxy-hydroxyphosphinyl)-L-leucyl-L-tryptophan (phosphoramidon), or a pharmaceutically, veterinary or agriculturally acceptable salt thereof.

As used herein, the term "alkyl" refers to a straight-chain or branched saturated hydrocarbon group and may have a specified number of carbon atoms. For example, C₁-C₆ as in "C₁-C₆alkyl" includes groups having 1, 2, 3, 4, 5 or 6 carbons in a linear or branched arrangement. Examples of suitable alkyl groups include, but are not limited to, methyl, ethyl, *n*-propyl, *i*-propyl, *n*-butyl, *i*-butyl, *t*-butyl, *n*-pentyl, 2-methylbutyl, 3-methylbutyl, 4-methylbutyl, *n*-hexyl, 2-methylpentyl, 3-methylpentyl, 4-methylpentyl, 5-methylpentyl, 2-ethylbutyl and 3-ethylbutyl.

As used herein, the term "alkenyl" refers to a straight-chain or branched hydrocarbon group having one or more double bonds between carbon atoms and may have a specified number of carbon atoms. For example, C₂-C₆ as in "C₂-C₆alkenyl" includes groups having 2, 3, 4, 5 or 6 carbon atoms in a linear or branched arrangement. Examples of suitable alkenyl groups include, but are not limited to, ethenyl, propenyl, isopropenyl, butenyl, pentenyl and hexenyl.

As used herein, the term "alkynyl" refers to a straight-chain or branched hydrocarbon group having one or more triple bonds between carbon atoms, and may have a specified number of carbon atoms. For example, C₂-C₆ as in "C₂-C₆alkynyl" includes groups having 2, 3, 4, 5 or 6 carbon atoms in a linear or branched arrangement. Examples of suitable alkynyl groups include, but are not limited to, ethynyl, propynyl, butynyl, pentynyl and hexynyl.

As used herein the term "halo" or "halogen" refers to fluorine (fluoro), chlorine (chloro), bromine (bromo) and iodine (iodo).

The term "alkyloxy" as used herein represents an alkyl group as defined above attached through an oxygen bridge. Examples of suitable alkyloxy groups include, but are not limited to, methoxy, ethoxy, *n*-propyloxy, *i*-propyloxy, *n*-butyloxy, *i*-butyloxy, t-butyloxy, *n*-pentyloxy and *n*-hexyloxy.

The term "alkylthio" as used herein represents an alkyl group as defined above attached through a sulfur bridge. Examples of suitable alkylthio groups include, but are not limited to, methyltlio, ethylthio, propylthio, *i*-propylthio, butylthio, *i*-butylthio, t-butylthio, pentylthio, hexylthio.

The term "carbocyclic ring" as used herein refers to a 3 to 10 membered ring or fused ring system, in which all of the atoms that form the ring are carbon atoms. The C₃₋₁₀ carbocyclic ring may be saturated, unsaturated or aromatic. Examples of suitable carbocyclic rings include, but are not limited to, cyclopropyl, cyclobutyl, cyclopentyl, cyclopentenyl, cyclohexyl, cyclohexenyl, phenyl, naphthyl and tetrahydronaphthyl.

The term "heterocyclic ring" as used herein refers to a 3 to 10 membered ring or fused ring system in which at least one of the atoms that form the ring is a heteroatom. Preferably the heteroatom is selected from nitrogen, oxygen, sulfur and phosphorus. The C₃₋₁₀ heterocyclic ring may be saturated, unsaturated or aromatic. Examples of suitable heterocyclic rings include, but are not limited to, benzoimidazolyl, benzofuranyl, benzofurazanyl, benzopyrazolyl, benzotriazolyl, benzothiophenyl, benzoxazolyl, carbazolyl, carbolinyl, cinnolinyl, furanyl, imidazoyl, indolinyl, indolyl, indolazinyl, indazolyl, isobenzofuranyl, isoindolyl, isoquinolyl, isothiazolyl, isoxazolyl, naphthpyridinyl, oxadiazolyl, oxazolyl, oxazoline, isoxazoline, oxetanyl, pyranyl, pyrazinyl, pyrazolyl, pyridazinyl, pyridopyridinyl, pyridazinyl, pyridyl, pyrimidyl, pyrrolyl, quinazolinyl, quinolyl, quinoxalinyl, tetrahydropyranyl, tetrazolyl, tetrazolopyridyl, thiadiazolyl, thiazolyl, thienyl, triazolyl, azetidinyl, aziridinyl, 1,4-dioxanyl, hexahydroazepinyl, piperazinyl, piperidinyl, pyrrolidinyl, morpholinyl, thiomorpholinyl, dihydrobenzoimidazolyl, dihydrobenzofuranyl, dihydrobenzothiophenyl, dihydrobenzoxazolyl, dihydrofuranyl, dihydroimidazolyl, dihydroindolyl, dihydroisooxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolyl, dihydroquinolinyl, dihydrotetrazolyl, dihydrothiadiazolyl, dihydrothiazolyl, dihydrothienyl, dihydrotriazolyl, dihydroazetidinyl, methylenedioxybenzoyl, tetrahydrofuranyl, and tetrahydrothienyl, and N-oxides thereof. Attachment of a heterocyclyl substituent can occur via a carbon atom or via a heteroatom.

As used herein, the term "aryl" is intended to mean any stable, monocyclic or bicyclic carbon ring of up to 6 atoms in each ring, wherein at least one ring is aromatic. Examples of such aryl groups include, but are not limited to, phenyl, naphthyl and tetrahydronaphthyl.

The term "heteroaryl" as used herein, represents a stable monocyclic or bicyclic ring of up to 6 atoms in each ring, wherein at least one ring is aromatic and at least one ring contains from 1 to 4 heteroatoms selected from the group consisting of O, N and S. Heteroaryl groups within the scope of this definition include, but are not limited to, acridinyl, carbazolyl, cinnolinyl, quinoxalinyl, pyrrazolyl, indolyl, benzotriazolyl, furanyl, thienyl, benzothienyl, benzofuranyl, quinolinyl, isoquinolinyl, oxazolyl, isoxazolyl, indolyl, pyrazinyl, pyridazinyl, pyridinyl, pyrimidinyl, pyrrolyl, tetrahydroquinoline.

Used compounds of the invention may be in the form of pharmaceutically, veterinary or agriculturally acceptable salts. Suitable pharmaceutically acceptable salts include, but are not limited to, salts of pharmaceutically acceptable inorganic acids such as hydrochloric, sulphuric, phosphoric, nitric, carbonic, boric, sulfamic, and hydrobromic acids, or salts of pharmaceutically acceptable organic acids such as acetic, propionic, butyric, tartaric, maleic, hydroxymaleic, fumaric, maleic, citric, lactic, mucic, gluconic, benzoic, succinic, oxalic, phenylacetic, methanesulphonic, toluenesulphonic, benezenesulphonic, salicyclic sulphanilic, aspartic, glutamic, edetic, stearic, palmitic, oleic, lauric, pantothenic, tannic, ascorbic and valeric acids.

Base salts include, but are not limited to, those formed with pharmaceutically acceptable cations, such as sodium, potassium, lithium, calcium, magnesium, ammonium and alkylammonium.

Basic nitrogen-containing groups may be quarternised with such agents as lower alkyl halide, such as methyl, ethyl, propyl, and butyl chlorides, bromides and iodides; dialkyl sulfates like dimethyl and diethyl sulfate; and others.

It will also be recognised that many compounds used in the invention possess asymmetric centres and are therefore capable of existing in more than one stereoisomeric form. The invention thus also relates to compounds in substantially pure isomeric form at one or more asymmetric centres eg., greater than about 90% ee, such as about 95% or 97% ee or greater than 99% ee, as well as mixtures, including racemic mixtures, thereof. Such isomers may be prepared by asymmetric synthesis, for example using chiral intermediates, or by chiral resolution.

A number of metal chelating agents and metalloprotease inhibitors useful in the present invention can be obtained commercially from speciality chemical companies. Those not commercially available can be synthesised from commercially available starting materials using reactions known to those skilled in the art.

For example, substituted 2,2-bipyridyls and 1,10-phenanthrolines may be obtained from suitable halogenated 2,2-bipyridyls or 1,10-phenanthrolines. For example, 2,2'-bipyridin-6,6'-dicarboxylic acid may be obtained from 6,6'-dibromo-2,2'-dipyridyl by halogen-metal exchange with butyl lithium, treatment with dry ice and acidification [Buhleier et. al., Chem. Ber., 1978, 111: 200-204]. Monosubstitution of a bipyridyl, for example with CH₂CHNH₂(CO₂H) at the 6 position, can be obtained by treatment of 6-methyl-2,2'-bipyridyl with N-bromosuccinimide followed by alkylation with N-protected-glycine ester. The protecting groups can then be removed by acid hydrolysis, (Imperiali B. and Fisher S.L., J. Org. Chem., 1992, 57: 757-759).

2,2'-Dipyridyls can undergo nucleophilic substitution at the C6 and C4 positions to introduce substituents. This reaction is more favorable when a halogenated dipyridyl is used as the starting material. For example an amine may be introduced at C6 and/or C6' by using 6-mono or di-halogenated 2,2'-dipyridyl and reacting this starting material with ammonia.

Bipyridyl-sulfonic acids can be prepared from 2,2'-bipyridyl by heating with either oleum (a solution of sulfur trioxide in concentrated sulfuric acid) or mercury (II) sulfate/concentrated sulfuric acid at 300°C.

Unsymmetrically substituted bipyridyls can be obtained from symmetrical bipyridyls, for example, 6'-methyl-2,2'-bipyridyl-6-carboxylic acid can be prepared from 6,6'-dimethyl-2,2'-bipyridyl by oxidation with selenium dioxide followed by treatment with silver nitrate (Al-Sayah et. al., European J. Org. Chem., 2004, 173-182).

Compounds of formulae (III) and (IV) can be prepared from commercially available amino acids, for example phenylalanine and tryptophan, using known coupling reactions with amino acid carboxylic acids or amine groups (Jones J., Amino Acid and Peptide Synthesis, Oxford Chemistry Press, 1992). Suitable protection and deprotection steps may be required as known in the art and shown in Jones, 1992, *Supra* or Green T.W. and Wutz P., Protecting Groups in Organic Synthesis, John Wiley & Son, 3rd Ed., 1999.

Thioureas of formula (V) may be prepared by reaction of a suitable benzamide with butyl lithium followed by thiophosgene. The resulting product can then be reacted with a suitable amine or amino acid as shown in Scheme 1.

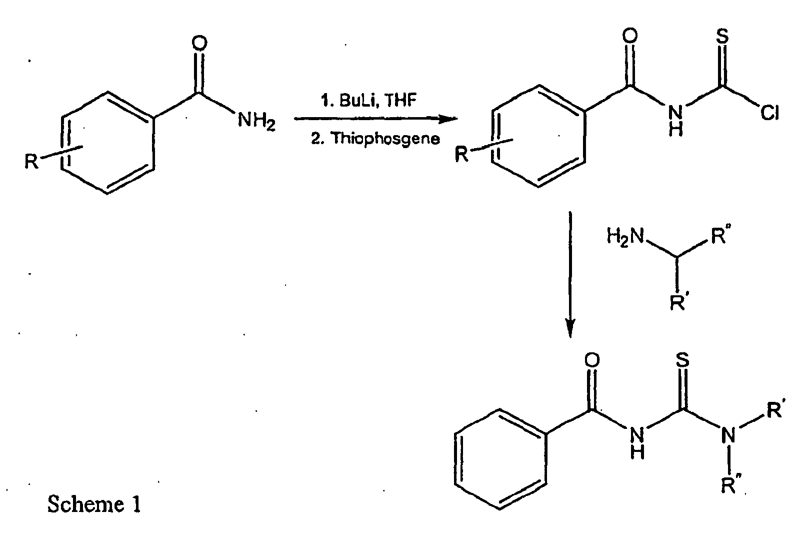

In the present specification, the term "ectoparasite" is taken to include any parasitic animal species that externally infests a host and that reproduces by egg laying. Preferred ectoparasites of the invention include a species from an order selected from the group consisting of Lepidoptera, Hemiptera, Orthoptera, Psocoptera, Hymenoptera, Isoptera, Coleoptera, Dictyoptera, Thysanoptera, Homoptera, Diptera, Anaplura, Malophaga, Siphonaptera and Arachnida.

Suitable ectoparasites that may be controlled using the methods of the present invention include:
(a) from the order of the lepidopterans (Lepidoptera), for example, *Adoxophyes orana, Agrotis ypsilon, Agrotis segetum, Alabama argillacea, Anticarsia gemmatalis, Argyresthia conjugella, Autographa gamma, Cacoecia murinana, Capua reticulana, Choristoneura fumiferana, Chilo partellus, Choristoneura occidentalis, Cirphis unipuncta, Cnaphalocrocis medinalis, Crocidolomia binotalis, Cydia pomonella, Dendrolimus pini, Diaphania nitidalis, Diatraea grandiosella, Earias insulana, Elasmopalpus lignosellus, Eupoecilia ambiguella, Feltia subterranea, Grapholitha funebrana, Grapholitha molesta, Heliothis armigera, Heliothis virescens, Heliothis zea, Hellula undalis, Hibernia defoliaria, Hyphantria cunea, Hyponomeuta malinel*/*us, Keiferia lycopersicella, Lambdina fiscellaria, Laphygma exigua, Leucoptera scitella, Lithocolletis blancardella, Lobesia botrana, Loxostege sticticalis, Lymantria dispar, Lymantria monacha, Lyonetia clerkella, Manduca sexta, Malacosoma neustria, Mamestra brassicae, Mocis repanda, Operophthera brumata, Orgyia pseudotsugata, Ostrinia nubilalis, Pandemis heparana, Panolis flammea, Pectinophora gossypiella, Phthorimaea operculella, Phyllocnistis citrella, Pieris brassicae, Plathypena scabra, Platynota stultana, Plutella xylostella, Prays citri, Prays oleae, Prodenia sunia, Prodenia ornithogalli, Pseudoplusia includens, Rhyacionia frustrana, Scrobipalpula absoluta, Sesamia inferens, Sparganothis pilleriana, Spodoptera frugiperda, Spodoptera littoralis, Spodoptera litura, Syllepta derogata, Synanthedon myopaeformis, Thaumatopoea pityocampa, Tortrix viridana, Trichoplusia ni, Tryporyza incertulas, Zeiraphera canadensis;*
(b) from the order of the hemipterans (Hemiptera), for example, *Aphis, Bemisia, Phorodon, Aeneolamia, Empoasca, Parkinsiella, Pyrilla, Aonidiella, Coccus, Pseudococcus, Helopeltis, Lygus, Dysdercus, Oxycarenus, Nezara, Aleyrodes, Triatoma, Psylla, Myzus, Megoura, Phylloxera, Adelges, Nilaparvata, Nephotettix or Cimwx spp.;*
(c) from the order of the orthopterans (Orthoptera), for example, *Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Schistocerca americana, Schistocerca peregrina, Stauronotus maroccanus, Schistocerca gregaria;*
(d) from the order of the psocopterans (Psocoptera), for example, *Peripsocus* spp.;
(e) from the order of the hymenopterans (Hymenoptera), for example, *Athalia rosae, Atta cephalotes, Atta sexdens, Atta texana, Hoplocampa minuta, Hoplocampa testudinea, Iridomyrmes humilis, Iridomyrmex purpureus, Monomorium pharaonis, Solenopsis geminata, Solenopsis invicta, Solenopsis richteri, Technomyrmex albipes;*
(f) from the order of the termites (Isoptera), for example, *Calotermes flavicollis, Coptotermes spp, Leucotermes flavipes, Macrotermes subhyalinus, Nasutitermes spp* such as *Nasutitermes walkeri, Odontotermes formosanus, Reticulitermes lucifugus, Termes natalensis;*
(g) from the order of the beetles (Coleoptera), for example, *Anthonomus grandis, Anthonomus pomorum, Apion vorax, Atomaria linearis, Blastophagus piniperda, Cassida nebulosa, Cerotoma trifurcata, Ceuthorhynchus assimilis, Ceuthorhynchus napi, Chaetocnema tibialis, Conoderus vespertinus, Crioceris asparagi, Dendroctonus refipennis, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus* , *Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllopertha horlicola, Phyllophaga sp., Phyllotreta chrysocephala, Phyllotreta nemorum, Phyllotreta striolata, Popillia japonica, Psylliodes napi, Scolytus intricatus, Sitona lineatus;*
(h) from the order Dictyoptera, for example, from the families *Polyphagidae, Bladberidae, Blattidae, Epilampridae, Chaetecsidae, Metallycidae, Mantoididae, Amorphoscelidae, Eremiaphilidae, Hymenopodidae, Mantidae* and *Empusidae;*
(i) from the order of the thrips (Thysanoptera), for example, *Frankliniella fusca, Frankliniella occidentalis, Frankliniella tritici, Haplothrips tritici, Heliothrips haemorrhoidalis, Scirtothrips citri, Thrips oryzae, Thrips palmi, Thrips tabaci;*
(j) from the order of the homopterans (Homoptera), for example, *Acyrthosiphon onobrychis, Acyrthosiphon pisum, Adelges laricis, Aonidiella aurantii, Aphidula nasturtii, Aphis fabae, Aphis gossypii, Aphis pomi, Aulacorthum solani, Bemisia tabaci, Brachycaudus cardui, Brevicoryne brassicae, Dalbulus maidis, Dreyfusia nordmannianae, Dreyfusia piceae, Dysaphis radicola, Empoasca fabae, Eriosoma lanigerum, Laodelphax striatella, Macrosiphum avenae, Macrosiphum euphorbiae, Macrosiphon rosae, Megoura viciae, Metopolophium dirhodum, Myzus persicae, Myzus cerasi, Nephotettix cincticeps, Nilaparvata lugens, Perkinsiella saccharicida, Phorodon humuli, Psylla mali, Psylla piri, Psylla pyricola, Rhopalosiphum maidis, Schizaphis graminum, Sitobion avenae, Sogatella furcifera, Toxoptera citricida, Trialeurodes abutilonea, Trialeurodes vaporariorum, Viteus vitifolii;*
(k) from the order of the dipterans (Diptera), for example, *Anastrepha ludens, Ceratitis capitata, Contarinia sorghicola, Dacus cucurbitae, Dacus oleae, Dasineura brassicae, Delia coarctata, Delia radicum, Hydrellia griseola, Hylemyia platura, Liriomyza sativae, Liriomyza trifolii, Lucilia Sp., Mayetiola destructor, Musca sp., Orseolia oryzae, Oscinella frit, Pegomya hyoscyami, Phobia antiqua, Phorbia brassicae, Phorbia coarctata, Rhagoletis cerasi, Rhagoletis pomonella;*
(l) from the order Anaplura, for example, *Pthirus pubis, Pediculus humanus capitus, Pediculus humanus humanus;*
(m) from the order Mallophaga, for example, from the genera *Bovicola, Damalania, Trichodectus* and *Menopon;*
(n) from the order of the siphonapterans (Siphonaptera), for example, *Ctenocephalides* or *Pulex* spp.
(o) from the order Arachnida, for example, *Ixodes holocyclus, Boophilus microplus, Rhipicephalus sanguineus, Sarcoptes scabiei* and *Dermatophagoides spp.*

It is preferred that the ectoparasite egg which is prevented from hatching by the present invention is selected from the group consisting of louse, flea, tick, fly and other biting or blood-sucking ectoparasite eggs. In a preferred embodiment, the ectoparasite egg is a louse egg, more preferably head louse egg. Lice are a parasite that feed on animal skin and blood and they deposit their digestive juices and faecal material into the skin. These materials, as well as the puncture wound itself, cause skin irritation and lesions from the resulting scratching, and can cause a serious infection with ganglionic inflammation. Lice are also vectors of certain diseases, such as exanthematic or epidemic typhus and recurrent fever. The adult female louse has a life span of about one month and can lay up to ten eggs a day. Lice that infect humans may include the species of crab louse *(Pthirus pubis)* and the separate species *Pediculus humanus* which is composed of two subspecies, *Pediculus humanus capitis* or head lice and *Pediculus humanus humanus* or clothing lice (Busvine, Antenna, 1993, 17: 196-201). The above subspecies of lice are closely related and are known to successfully interbreed (Busvine, Cutaneous Infestations and Insect Bites, 1985, 163-174).

The head louse *Pediculus humanus* var. *capitis,* is a host-specific ectoparasite that lives exclusively on human heads and feeds via sucking blood from the scalp. Following a blood meal, mature adult female lice will lay up to 10 eggs close to the scalp over a 24 hr period. The eggs are attached firmly to the hair shaft via a glue. Seven to ten days post laying depending on temperature and humidity, the eggs will hatch and the newly emerged nymphs begin to feed. The nymphs progress through three moults (1^{st} instar, 2^{nd} instar, 3^{rd} instar) with each moult taking between 3-5 days to complete. Following the final moult the adult male or female emerges with mating taking place as early as two days later. Within hours of feeding, eggs will be produced and the cycle continues. The entire life cycle from egg to egg takes approximately 20-30 days to complete depending on conditions of warmth and humidity. Following egg hatching the egg shell remains attached to the hair shaft and will gradually move away from the scalp as the hair lengthens. Hatched eggs (nits) are relatively easily detected due to their refractive nature appearing white under artificial light, in contrast unhatched eggs are a light pale brown in color enabling them to blend in to most hair colors and therefore making them more difficult to detect.

In one embodiment of the present invention, the methods and compositions used in the invention are to cure a subject of lice by inhibiting hatching of louse eggs. The present applicants have identified metal chelating agents and metalloprotease inhibitors as an effective agent for inhibiting ectoparasite louse egg hatching. The use of metal chelating agents or metalloprotease inhibitors for inhibiting ectoparasite louse egg hatching has the advantage of preventing breeding cycles of ectoparasites thereby controlling ectoparasite infestation.

The term "metalloprotease" as used herein is taken to refer to a protease involved in ectoparasite egg hatching, wherein the protease has an active metal ion that acts as a catalyst. Preferably, the metalloprotease contains a zinc ion that participates in catalysis by polarizing a water molecule to attack a substrate-peptide bond. More preferably, the metalloprotease is sensitive to metal chelating agents that are capable of blocking their activity. The metalloprotease preferably is involved in inducing egg hatching by acting on the operculum of an egg to facilitate egg hatching. Suitable metalloprotease involved in ectoparasite egg hatching can include endoproteases (enzymes that cleave within the peptide chain) and exoproteases (enzymes that cleave amino acid(s) from the termini of peptides). Exoproteases can further be categorised as carboxyproteases (which cleave amino acid(s) from the C terminus) or aminopeptidase (which cleave amino acids from the N terminus). Metallo-carboxyproteases require a bivalent cation (usually Zn²⁺) for activity, while aminopeptidases are generally classified according to their dependence on metal ions (Zn²⁺ or Mg²⁺). They exist in both free and membrane-bound forms and favour activity at high (8-10) pH. One method of detecting metalloproteases associated with egg hatching can involve collecting either the fluid surrounding the developing embryo at the time of egg hatching or by washing the empty egg shells shortly after egg hatching and analyzing the sample for the presence of proteases using gelatine substrate SDS-PAGE analysis. Having shown the presence of proteolytic activity from the sample it is then possible to incubate the sample in the presence of a metalloprotease inhibitor, for example, 1,10-phenantholine, and then reanalyze the treated sample to determine if the activity of the proteases extracted from the egg have been inhibited. Having shown inhibition of the activity of the metalloprotease(s) obtained from the hatched egg, it is then possible to expose unhatched eggs to the same inhibitor and assess whether inhibition of egg hatching occurs. Metalloproteases involved in egg hatching may also be identified by identification of a gene encoding a metalloprotease, silencing that gene and showing that the egg is unable to hatch by methods known to those skilled in the art.

The phrase "inhibiting hatching of an ectoparasite egg" as used herein is taken to mean that hatching of an ectoparasite egg is prevented. In the present invention an ectoparasite egg is exposed to a metal chelating agent or a metalloprotease inhibitor that is capable of preventing egg hatching when compared to an untreated ectoparasite egg. Egg hatching is characterised by the hatchflap or operculum of an egg opening and shortly thereafter the emergence of a nymph. In the case of lice, the head appears first followed by the thorax to which the legs are attached. Finally, the abdomen emerges and the nymph moves free from the egg. Egg hatching is taken to exclude damage or accidental breakage of an eggshell.

Preferably, the metal chelating agent or metalloprotease inhibitor is a compound capable of inhibiting egg hatching when it is applied to the egg at any time between laying and hatching.

The ectoparasite egg is preferably present on, but not limited to, a host organism, such as on the skin, hair, coat or fleece of an animal or head hair of a human. In alternative embodiments of the invention the ectoparasite egg may be present on host animals including humans, host plants including cereal crops, fruit trees, cotton, oil seed crops, ornamental plants, flowers, vine crops, root crops, pasture plants and vegetables, or other breeding sites, such as, but not limited to, houses and buildings, enclosures for domestic and farming animals, carpets, blankets, curtains and furniture.

According to the present invention, the ectoparasite egg may be exposed to a metal chelating agent or a metalloprotease inhibitor by any suitable means. A person skilled in the art will appreciate that these means may vary widely, depending upon whether the inhibitor is to be applied to a host, such as a plant or animal or various other breeding sites, and depending on the nature and type of ectoparasite targeted. Suitable means for exposing ectoparasite eggs present on animals to metal chelating agents or metalloprotease inhibitors, include, but are not limited to, direct topical application, such as by dipping or spraying, implants, delayed release formulations or devices. Where the invention is applied to humans, formulations suitable for topical application include but are not limited to sprays, aerosols, shampoos, mousses, creams and lotions, and formulations suitable for internal application include but are not limited to tablets, capsules or liquid formulations. In some situations parenteral administration by injection may be the most suitable means of treatment for humans or animals. Where the metal chelating agent or metalloprotease inhibitor is to be applied to plants, suitable means include but are not limited to sprays, dusts, pellets, or aerosols. The method of the invention also encompasses the concurrent or successive use of two or more metal chelating agents or metalloprotease inhibitors or the use of one or more metal chelating agents and/or metalloproteases in conjunction concurrently or successively with other known agents that control ectoparasites.

In yet another aspect of the invention, the methods and compositions may include other ectoparasiticides that control hatching, nymphs or adult ectoparasites. For example, suitable ectoparasiticides which may be used in conjunction, either simultaneously or separately, with the metal chelating agents or metalloprotease inhibitors of the present invention include organophosphates such as malathion, synthetic pyrethroids (cypermethrin, deltamethrin) insect growth regulators, including juvenile hormone analogues, chitin synthesis inhibitors and triazine derivatives, insecticidal bacterial toxins, chlorinated hydrocarbons (DDT, endosulfan) or insecticidal agents described in EP 0191236, US 5,288,483 and US 6,727,228. Other useful insecticides include dimethicone copolyols, such as those described in US 6,663,876 and US 6,607,716, which have low toxicity. The advantage of such a combination is that only one application may be required to control the ectoparasite over all of its life cycle.

The metal chelating agent or the metalloprotease inhibitor may be applied to the hair or skin of a host, preferably in a region that is infested with an ectorparasite. The ectoparasite infestation may preferably be due to ectoparasites selected from the group consisting of lice, fleas, ticks, flies and other biting or blood-sucking ectoparasites, and combinations thereof. Most preferably, the ectoparasite infestation is due to lice. The metal chelating agent or the metalloprotease inhibitor may be applied topically in the form of ointments, aqueous compositions including solutions and suspensions, creams, lotions, aerosol sprays or dusting powders.

The term "effective amount" means a concentration of at least one metal chelating agent or at least one metalloprotease inhibitor sufficient to provide treatment or prevention of ectoparasite infestation in a host. The effective amount of a metal chelating agent or metalloprotease inhibitor used in the methods of the present invention may vary depending on the host and the type and level of ectoparasite infestation. The metal chelating agent or metalloprotease inhibitor is preferably applied to the scalp of a person suffering from head lice infestation and are left on the treated person for a period of time to prevent hatching of the louse eggs. Preferably the period of time is between 5 and 15 minutes. The metal chelating agent or metalloprotease inhibitor is preferably used at a concentration of between about 0.0001mM to 1M, preferably 0.01mM and 100mM, more preferably in the range of 0.1mM and 30mM. The effective amount depends on the metal chelating agent or metalloprotease used. However, some dipyridyl compounds may suitably applied in the range of 5mM to 15mM, especially at a level of about 10mM. Since a significant number of mammalian proteases require zinc for their activity and may be effected by metal chelating agents and/or metalloprotease inhibitors, it would be necessary to ensure that the metal chelating agent or metalloprotease inhibitor was used in a safe and effective amount and is preferably specifically targeted to ectoparasite eggs.

The host treated by the methods of the invention may be selected from, but is not limited to, the group consisting of humans, sheep, cattle, horses, pigs, poultry, dogs and cats. The methods of treatment or prevention of the present invention may be applicable to plants and or other breeding sites of ectoparasites. Plants treated by the methods of the invention are preferably selected from the group consisting of cotton, oil seed crops, ornamental plants, flowers, fruit trees, cereal crops, vine crops, root crops, pasture plants and vegetables.

The compositions used in the present invention may be formulated as solutions and emulsions. Suitable excipients, such as emulsifiers, surfactants, stabilizers, dyes, penetration enhancers and anti-oxidants may also be present in the compositions. Suitable carriers that may be added in the compositions can include, water, salt solutions, alcohols, polyethylene glycols, gelatine, lactose, magnesium sterate and silicic acid. The compositions may include sterile and non-sterile aqueous solutions. The compositions are preferably in a soluble form and the metal chelating agent or metalloprotease inhibitor are preferably, diluted in a soluble sterile buffered saline or water solution. The compositions can also be formulated as suspensions in aqueous, non-aqueous or mixed media. Aqueous suspensions may further contain substances that increase the viscosity of the suspension and may also contain stabilizers. The solutions may also contain buffers, diluents and other suitable additives. The compositions can include other adjunct components that are compatible with the activity of the metal chelating agent or metalloprotease inhibitor. The compositions of the present invention may be formulated and used as foams, emulsions, microemulsions, shampoos, mousses, creams and jellies. The formulations of the above compositions described would be known to those skilled in the field of ectoparasiticides.

In a preferred embodiment, the composition comprises a metal chelating agent at a concentration of about 0.0001mM to 1M, preferably between 0.1mM to 100mM, more preferably in the range of 0.1mM to 30mM. Compositions containing some metal chelating agents or metalloprotease inhibitors, for example, the dipyridyl compounds, may preferably contain between 5 and 15mM of compound, especially at a level of about 10mM.

The invention also provides a method of identifying a compound which inhibits hatching of an ectoparasite egg, the method comprising assessing the ability of the compound to inhibit a metalloprotease present in the ectoparasite egg.

The effect of the compound on the activity of the metalloprotease may be assessed in a number of ways, however, in general the assessment preferably involves comparison of metalloprotease enzyme activity in the presence and absence of the test compound. One method of detecting metalloproteases associated with egg hatching can involve collecting either the fluid surrounding the developing embryo at the time of egg hatching or by washing the empty egg shells shortly after egg hatching and analyzing the sample for the presence of proteases using gelatine substrate SDS-PAGE analysis. Having shown the presence of proteolytic activity from the sample it is then possible to incubate the sample in the presence of a metalloprotease inhibitor, for example, 1,10-phenantholine, and then reanalyze the treated sample to determine if the activity of the proteases extracted from the egg have been inhibited. Having shown inhibition of the activity of the metalloprotease obtained from the hatched egg, it is then possible to expose unhatched eggs to the same inhibitor and assess whether inhibition of egg hatching occurs. Metalloproteases involved in egg hatching may also be identified by identification of a gene encoding a metalloprotease, silencing that gene and showing that the egg is unable to hatch by methods known to those skilled in the art.

In a preferred embodiment the method further comprises testing the compound in a biological ectoparasite egg hatching assay.

A suitable biological ectoparasite egg hatching assay preferably comprises exposing a control sample of ectoparasite eggs to a control buffer solution whilst at the same time exposing a test sample of ectoparasite eggs to a solution comprising a test compound.

A compound that is effective in inhibiting ectoparasite egg hatching is identified when egg hatching is observed in the control sample and a lower level of hatching is observed in the test sample. In the preferred biological egg hatching assay of the present invention, the ectoparasite eggs are selected from the group consisting of louse, flea, tick, fly and other biting or blood-sucking ectoparasite eggs. In a preferred embodiment the sample of ectoparasite eggs are lice eggs. Preferably, the egg samples (control and test samples) used are no more than post 6-7 days after being laid. Most preferably, the egg samples used are no more than 1 day after being laid.

The control buffer solution may include, but is not limited to, sterile phosphate buffered saline or water. The compound tested is preferably a metal chelating agent and/or a metalloprotease inhibitor. In the biological egg hatching assay egg hatching is observed when the hatchflap or operculum of the egg opens and shortly thereafter the nymph begins to emerge. In the case of lice, the head appears first followed by the thorax to which the legs are attached. Finally, the abdomen comes out and the nymph moves free from the egg. In the case of head lice, the eggshell then remains cemented to the hair shaft.

In another aspect of the invention there is provided a use of at least one metal chelating agent according to formula (Ia) in the manufacture of a composition for inhibiting hatching of an ectoparasite egg or for treating or preventing ectoparasite infestation.

In yet another aspect of the invention there is provided use of at least one metalloprotease inhibitor according to formula (Ia) in the manufacture of a composition for inhibiting hatching of an ectoparasite egg or for treating or preventing ectoparasite infestation.

Also encompassed by the present invention are agents comprising at least one metal chelating agent and/or at least one metalloprotease inhibitor according to formula (Ia), for inhibiting hatching of an ectoparasite egg or for treating or preventing ectoparasite infestation.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The invention will hereinafter be described by way of the following non-limiting Figures and Examples.

### EXAMPLES

### Example 1 -assessment of the mechanism of lice egg hatching:

The mechanism of lice egg hatching was assessed under a dissecting microscope. Female clothing lice were fed for half an hour on a rabbit before being transferred to a petri dish containing human hair. The petri dish was then placed in an incubator at 32°C; 42% relative humidity. Within 5 hours of feeding the female lice begin to lay their eggs. Each female lays up to 10 eggs at a sitting. The eggs develop over the next 7-9 days. Within the last 12 hrs prior to hatching the following changes were observed. The eyes of the developing embryo could be clearly detected inside the egg with the developing embryo orientated so that it has its head is adjacent to the hatch flap or operculum. The embryo can be observed moving within the egg. Hatching takes place when the operculum opens and shortly thereafter the embryo begins to emerge. The head appears first followed by the thorax to which the legs are attached. Finally, the abdomen comes out and the nymph moves free from the egg that remains cemented to the hair. There are no obvious structures associated with the head of the newly emerged nymph visible under light microscopy, that would facilitate hatching (ie no egg tooth is present). This observation suggests that while physical movement of the nymph within the egg probably contributes to egg hatching, other specific biochemical events are involved.

### Example 2 -detection of protease activity in lice egg extracts:

Within 12 hours of hatching 50 body lice eggs *(Pediculus humanus humanus)* were removed from the hair and placed in a 1 ml eppendorf tube. 20 µL of distilled water was added to the unhatched eggs and the preparation incubated for 30 minutes at 32°C. The 20 µL was recovered and stored at -70°C. A number of other samples were also collected as described. Hair samples from which unhatched lice eggs had been removed were also collected and incubated as described above. In addition, a sample of unhatched eggs and a sample of hair from which lice eggs had been removed were collected 7 days post laying (within 24 hrs of egg hatching). Both samples were washed in 10 mls of a 1% solution of sodium hypochlorite for 1 minute followed by a 5 x 1 minute washes in 25mls in distilled water to remove the hypochlorite. These samples were then incubated in 20µL of distilled water as described above. In addition, a group of 25 lice eggs that were within 24 hours of hatching, were pretreated with 1% sodium hypochlorite, washed as described above and left to hatch. Within 1-2 hours after hatching the empty egg shells were incubated in 20µL of distilled water as described above, the washings collected from the hatched egg shells and stored at -70°C. All of the extract samples were then freeze-dried overnight. The freeze-dried samples were then resuspended in 15µL of non-reducing SDS sample buffer, centrifuged at 10,000g for 2 minutes and the entire 15µL loaded on to 10% gelatine substrate SDS-PAGE gels. Gels were run at 4°C for 10 minutes at 10mA followed by a further 25 minutes at 15mA per gel. They were then incubated for 2 x 20 minutes in a 2.5% Triton-X 100 solution followed by a three hour incubation in 0.1M Tris/HCl containing 1mM CaCl₂ pH 8.0. Activity was detected as clear areas on the gel the result of protease activity degrading the gelatine within the gel.

The results from these studies indicated that proteolytic activity was present in a number of different preparations as analysed using substrate SDS-PAGE. Protease activity was detected in the washings obtained from unhatched lice eggs within 12 hours of hatching (Figure 1, lane 1). This activity was in the higher molecular weight region of the gel. When hair samples that had had lice eggs removed were analysed on gelatine substrate SDS-PAGE a significant amount of protease activity was detected (Figure 1, lane 2). The most likely explanation for this activity was that it was of maternal origin being produced at the time of laying. Treatment of hair samples with sodium hypochlorite completely removed the contaminating proteases (Figure 1, lane 3). In addition treatment of unhatched eggs was also able to remove this protease activity (Figure 1, lane 4). It was therefore decided to treat all eggs prior to hatching with 1% Na Hypochlorite as described above in order to remove these maternal proteases. Analysis of freshly hatched egg shells (Egg Shell Washings) indicated the presence of two high molecular weight species (Figure 1, lane 5). Note only 25 lice eggs were used in this collection, possibly contributing to the lower level of protease activity. These results demonstrate the presence of protease activity directly associated with freshly hatched lice eggs.

In conclusion the hatching process in lice was studied by light microscopy. Egg hatching appears to be associated with physical activity of the developing nymph within the egg. However, the lack of any specialised structures for piercing or loosening the hatch flap or operculum indicates that the hatching process may also involve a biochemical component. While highly active proteases were detected around the time of egg hatching in lice the primary source of these proteases appears to be of maternal origin. Removal of this activity prior to egg hatching was achieved using sodium hypochlorite with the lice progressing through to successfully hatch. Subsequent analysis of the ESW from freshly hatched lice indicated the presence of a limited number of protease species that were further investigated as targets for inhibiting egg hatching in lice.

### Example 3: characterisation of proteases in egg shell washings:

In order to evaluate the potential of lice hatching proteases in the egg shell washings as targets for inhibiting egg hatching it was first necessary to characterize the nature of the hatching proteases. Inhibitors of the 4 major classes of proteases were used to classify the proteases in the ESW.

10% SDS-PAGE gelatine substrate gels were loaded with freeze dried egg shell washings from 100 lice eggs that had been resuspended in 50 µl of non-reducing sample buffer with samples run at 10 µl per lane. Gels were run at 4°C for 10 minutes at 10 mA per gel followed by a further 25 minutes at 15 mA per gel. Gels were then cut into strips and each strip incubated for 2 x 20 minutes in a 2.5% Triton-X 100 solution containing a specific inhibitor. The inhibitors used were the serine protease inhibitor PMSF (5mM), the metalloprotease inhibitor 1,10-phenanthroline (10mM), the aspartic protease Pepstatin (5µM) and the cysteine inhibitor E-64 (10µM). The gel strips were then incubated in 0.1M Tris/HCl containing 1mM CaCl₂ pH 8 containing the different protease inhibitors for 3 hrs at 37°C, before being stained in Coomassie blue and destained as previously described. In contrast to Figure 1, lane 5 that shows a predominance of proteolytic activity around 25-30 kDa (refer to Figure 2 brackets). Subsequent analysis of numerous preparations of ESW indicated that this triplet of proteolytic activity around 25-30 kDa was highly reproducible.

The results from the inhibitor studies indicate a significant reduction in protease activity following treatment with 1,10-phenanthroline (lane 2 bracketed region). No reduction in protease activity of the ESW was observed when the serine protease inhibitor PMSF or the cysteine protease inhibitor E64 was used. In addition, the aspartic inhibitor pepstatin did not show any reduction in protease activity (data not shown).

### Example 4: development of an in vitro bioassay for measuring lice egg hatching:

To evaluate the potential effects of protease inhibitors on lice egg hatching it was necessary to develop a reliable *in vitro* bioassay. Male and female clothing lice were fed on a rabbit as previously described. Female and male adult lice in a ratio of 3:1 were then transferred to a clean petri dish containing nylon cloth approximately 3 x 3 cm² and left for 12 hrs at 32°C. During this period the female lice laid their eggs and attached them to the woven cloth. All lice would then be removed and the eggs permitted to incubate for the following 5 days. On Day 6 the cloth containing the eggs would be placed for 1 minute in a 1% sodium hypochlorite solution and then washed extensively. The eggs would then progress through to their final stages of development and hatch. In untreated control eggs a reliable average percentage hatch of between 85-95 percent was obtained using the *in vitro* egg hatch assay. It was subsequently found that for the egg hatching assay it was not necessary to pre-treat the lice eggs with sodium hypochlorite.

### Example 5: identification of compounds that can inhibit the activity of lice hatching proteases:

### (a) Testing of protease inhibitors using Lice egg-hatching bioassay.

Having refined a bioassay for measuring egg hatching in lice, the next phase of the research was to use this bioassay as a means of testing the effects of different protease inhibitors on egg hatching.

Lice eggs were laid onto cloth as described above. Five days post laying the cloth containing lice eggs was removed and immersed in a 1% sodium hypochlorite solution before being washed extensively in distilled water and blotted dry on tissue paper. Lice eggs were counted under a dissecting microscope and the cloth cut into batches of between 10-30 eggs with 3-5 replicates used per treatment. The cloth containing lice eggs was then immersed in a protease inhibitor solution for a period of between 2-10 minutes, placed on tissue paper for 1 minute to dry before being transferred to a clean petri dish and incubated until hatching. The eggs were observed at regular time intervals for evidence of eggs hatching over the next 1-2 days by which time the control eggs had hatched. Protease inhibitor solutions were typically prepared as stock solutions and added fresh at the appropriate concentration. Specifically stock solutions were prepared as follows: 1,10-phenanthroline (200mM in methanol) and Bestatin (5mg/ml in methanol). In addition, the equivalent levels of the solvent were added to the non-inhibitor containing controls eggs to test for any buffer alone effects. Percentage hatch inhibition was calculated as the percentage reduction in egg hatch compared to the untreated control. The untreated control was assigned a percentage hatch of 100%.

The addition 1,10-phenanthroline, a metal chelating agent and a metalloprotease inhibitor significantly inhibited egg hatching in lice at 10mM while at 1mM the level of inhibition was approximately 30% compared to that of the controls (refer to Figure 3). Bestatin, a metal chelating agent and an inhibitor of metalloproteases and more specifically aminopeptidase M and N, was also able to significantly inhibit lice egg hatching at 5mM (Figure 4).

These results provide data on the effect of specific metal chelating agents and metalloprotease inhibitors on egg hatching in lice. It was however noted that when either 1,10-phenanthroline or Bestatin were added within 24 hours of hatching, variable inhibition of egg hatching was observed (data not shown). This variability in hatch inhibition could be due to a number of factors that relate to the specific developmental stage of the louse. Furthermore these studies indicated that it is very difficult to predict the exact time of egg hatch and therefore the choice of a single time point in which to treat the eggs may be problematic when assessing the effects of a specific inhibitor on egg hatch. The *in vitro* assay system was therefore modified to account for this variability in lice development.

### (b) Time course experiment using in the in vitro hatching assay.

A series of time-course experiments was conducted as a means of assessing inhibitors of lice egg hatching. Eggs were laid onto cloth as previously described and then at 24 hr intervals an inhibitor was added to a new group of eggs for eggs up to 120 hrs post laying. The eggs were then incubated at 28°C for a further 8 days to permit egg hatching. This method of assaying inhibitors more closely mirrors the field situation where lice eggs will be at various stages of development.

The results of these studies are shown in Table 1. Significant inhibition by 1,10-phenanthroline was demonstrated at varying concentrations over the course of lice hatching. A degree of concentration dependence was also observed with the inhibitory effects of 1,10-phenanthroline. The results indicate that time-course experiments provide a more reliable means of assessing the effects of specific inhibitors on lice egg hatching. The addition of Bestatin resulted in significant inhibition, but only when applied approximately 24 hrs prior to egg hatch.

**Table 1. Percent inhibition of egg hatching following treatment with different concentrations of 1,10-phenanthroline and 5mM Bestatin at 24 hr intervals post egg laying.**

| | **Time post egg laying (hr)** | | | | | |
|---|---|---|---|---|---|---|
| **Inhibitor** | **24 hr** | **48 hr** | **72 hr** | **96 hr** | **120 hr** | **144 hr** |
| **10 mM 1,10-phenanthroline** | 100 | 100 | 100 | 100 | 100 | 100 |
| **5 mM 1,10-phenanthroline** | 100 | 100 | 100 | 100 | 93 | 96 |
| **2.5 mM 1,10-phenanthroline** | 100 | 100 | 85 | 85 | 89 | 60 |
| **5 mM Bestatin** | - | - | - | - | - | 53 |

Results from the above studies indicate that lice hatching enzymes are proteases of the metallo class as judged by the ability of metal chelating agent and metalloprotease inhibitor 1,10-phenanthroline to inhibit their activity. Furthermore this compound was able to significantly inhibit egg hatching in lice at all time points examined with some evidence of a dose dependent effect particularly when eggs were treated with the lower concentrations around the time of hatching. 1,10-phenanthroline exerts its effects through its ability to chelate metal ions, preferably zinc and thereby inhibiting zinc dependent proteases.

The data for Bestatin also indicated that the compound could partially inhibit lice egg hatching when administered to eggs in the late developmental stage. Bestatin is a cyclic compound comprising an aryl ring substituted with a substituent containing an amine, a hydroxy group, an amide and a carboxylic acid group. Bestatin is an antibiotic of microbial origin, which is used for treating various forms cancer including nonlymphocytic leukemia and also different forms of solid tumors including, lung, stomach, bladder, head, neck and oesophagus where it is used under the name of ubenimex. It can be administered with low toxicity to cultured cells, intact animals and humans. While Bestatin is normally used as an inhibitor of purified proteases at micromolar concentrations (10µM and 130µM) the data obtained thus far show it being effective at 5mM. This result may be due to a number of factors including the ability of Bestatin to penetrate the egg and its specificity for the lice hatching proteases.

### Example 6: screening of protease inhibitors to inhibit lice egg hatching:

Lice eggs were laid onto cloth as described in Example 4. A series of time-course experiments were set-up as described in Example 5(part (b)). A considerable number of commercially available protease inhibitors/metal chelators were tested in the lice hatching assay to determine the effect of individual protease inhibitors on lice egg hatching (Table 2). Percentage hatch inhibition was calculated as the percentage reduction in egg hatching compared to the untreated control. The untreated control was assigned a percentage hatch of 100%.

**Table 2. Percentage inhibition of lice egg hatching following treatment with various protease inhibitors. The percentage inhibition refers to the maximum egg hatch obtained over the time-course of the experiment.**

| **Number** | **Inhibitor** | **%Inhibition*** |
|---|---|---|
| 1 | 1-10 phenanthroline (10mM) | 100 |
| 2 | 2,2-dipyridyl (10mM) | 100 |
| 3 | 6,6'-Dimethyl-2,2'-dipyridyl (10mM) | 100 |
| 4 | 5,5'-Dimethyl-2,2'-dipyridyl (10mM) | 100 |
| 5 | Captopril (23mM) | 27 |
| 6 | D-L Thiorophan (1mg/ml) | 20 |
| 7 | Phosphoramidon (5mM) | 41 |
| 8 | Actinonin (5mM) | 0 |
| 9 | Bestatin (5mM) | 58 |
| 10 | NitroBestatin (1mg/ml) | 0 |
| 11 | Amastatin (5mM) | 26 |
| 12 | Leuhistin (5mM) | 0 |
| 13 | Ebelactone (1, 2 and 5mM) | 0 |
| 14 | L-leucinthiol (5mM) | 0 |
| 15 | Fumagillin (5mM) | 0 |
| 16 | Carboxypeptidase inhibitor (5mM) | 26 |
| 17 | N-CBZ-PRO-LEU-GLY Hydroximate (10mM) | 0 |
| 18 | Tetracycline (5mg/ml) | 89 |
| 19 | Doxycycline (5mg/ml) | 69 |
| 20 | Minocycline (5mg/ml) | 55 |
| 21 | GM 1489 | 0 |
| 22 | GM6001 | 0 |
| 23 | Inhibitor IV | 0 |
| 24 | Chlorhexidine dihydrochloride | 0 |

| | | |
|---|---|---|
| *The percentage inhibition of egg hatching of the different protease inhibitors has been calculated relative to the appropriate controls and represents the maximum egg hatch inhibition observed. | | |

A number of protease inhibitors were shown to markedly inhibit lice egg hatching. The most effective inhibitors tested included metal chelating agents and metalloptotease inhibitors such as 1-10 phenanthroline, 2,2-dipyridyl and 6,6'-Dimethyl-2,2'-dipyridyl, 5,5'-dimethyl-2,2'-dipyridyl (100% inhibition at 10mM each). Bestatin, a metalloprotease inhibitor was also able to significantly inhibit egg hatching (58% at 5mM).

Naturally derived matrix metalloprotease (MMP) inhibitors and metal chelating agents that are tetracyclic compounds in which one ring is an aryl ring and wherein the tetracyclic structure is substituted with a number of hydroxy groups, carbonyl groups, an amine and an amide, included: Tetracycline (89% inhibition at 5mg/ml), Doxycycline (65% inhibition at 5mg/ml) and Minocycline (55% at 5mg/ml). These metal chelators showed inhibitory activity towards egg hatching, however the results for these compounds were more variable in magnitude and appeared to be time dependent. The overall usefulness of the hydroxamate inhibitors may be limiting due to the drive to reduce the use of antibiotics in the general environment. These results indicate that MMP inhibitors may also exert an inhibitory effect on lice egg hatching. Other protease inhibitors that were tested included EDTA at 100mM, EGTA at 10mM and Triethanolamine at 5%. The results indicated that these inhibitors did not appear to have an effect on egg hatching at the concentrations used (results not shown).

### Example 7: effect of washing eggs post treatment with 1-10 phenanthroline:

An experiment was undertaken to determine whether washing of the eggs would effect the inhibitory activity of 1,10-phenanthroline (Table 3). A control group (5% methanol) was also set up. Percentage hatch inhibition was calculated as the percentage reduction in egg hatch compared to the untreated control. The untreated control was assigned a percentage hatch of 100%. The results from this experiment indicate that 1,10-phenanthroline is still highly efficacious at inhibiting lice egg hatching following washing of eggs in water. In later stage eggs that are approaching egg hatch (day 5) the effects appear to reflect a concentration dependence similar to that observed when lower concentrations of the inhibitor were used. It was also noted that a proportion of eggs treated with 1,10-phenanthroline had embryos that appeared to develop normally yet failed to hatch.

**Table 3. Percent inhibition of egg hatching following treatment with 10mM 1-10 phenanthroline at 24 hr intervals post egg laying in lice. Lice eggs were treated with inhibitor for 10 minutes and left unwashed or treated and washed for 1 minute and then left to hatch.**

| | Time post laying (hr) | | | | |
|---|---|---|---|---|---|
| | 24 hr | 48 hr | 72 hr | 96 hr | 120 hr |
| Treated/not washed | 100 | 100 | 100 | 100 | 100 |
| Treated/was washed | 100 | 100 | 100 | 97 | 62 |

### Example 8: inhibition of hatching of head lice eggs with 1-10 phenanthroline:

Tests were carried out to determine if metal chelating agent and metalloprotease inhibitor 1,10-phenanthroline could inhibit head lice egg (*Pediculus humanus capitus*) hatching as opposed to body lice. Head lice eggs were obtained by placing groups of both 1-2 adult male and 6-8 adult female head lice in separate wells in a 24 well petri dish containing cotton cloth. The petri dish was transferred to a humid incubator at 32°C, 70% RH for 12 hours to permit the female lice to lay their eggs. After 12 hours, all adult lice were removed from the petri dish wells and a series of time-course experiments conducted. A group of eggs (24 hr old) was treated for 10 minutes with 200µl of a 10mM solution of 1,10-phenanthroline. A control (ie no inhibitor treatment) group of eggs was also included. The eggs were removed from the inhibitor, blotted dry on tissue paper, placed at 32°C, 70% RH and left to hatch. A second group of eggs, (48 hr old) were treated as previously described and also left to hatch. This process was repeated at 24 hr intervals on head lice eggs up to 120hr post laying. This method of assaying inhibitors more closely mirrors the field situation where lice eggs will be at various stages of development on the head and permits the inhibitory effects to be observed on these different stages of the parasite.

The results from the above studies indicate that 1,10-phenanthroline can significantly inhibit egg hatching in head lice (Table 4).

**Table 4. Percent inhibition of egg hatching following treatment with 10mM 1,10-phenanthroline at 24 hr intervals post egg laying in lice relative to the control.**

| | Days post laying | | | | |
|---|---|---|---|---|---|
| | 1 | 2 | 3 | 4 | 5 |
| Treated | 100 | 87 | 88 | 100 | 100 |

These results strongly suggest that body lice are an effective model for assaying the effects of protease inhibitors in egg hatching of head lice.

### Example 9: inhibition of lice egg hatching with metal chelators:

Experiments were conducted using two metal chelating agents that can act as metalloprotease inhibitors to determine their effects on lice egg hatching. These compounds were tested in the standard lice assay to determine their ovicidal effects (refer to example 5 and 6 on methods used to test inhibitors). The following metal chelating agents were evaluated: 2,2'-dipyridyl and 6,6'-Dimethyl-2,2'-dipyridyl. The results of this study are shown in Tables 5 and 6.

**Table 5. Results of egg hatching following treatment with 2,2'-dipyridyl at 24 hr intervals post egg laying. The results are indicated for: N (number of eggs per replicate), H (number of eggs successfully hatched) and Ph (number of eggs partly hatched).**

| Replicates | 24hr | | | 48hr | | | 72hr | | | 96hr | | | 120hr | | |
|---|---|---|---|---|---|---|---|---|---|---|---|---|---|---|---|
| | N | H | Ph | N | H | Ph | N | H | Ph | N | H | Ph | N | H | Ph |
| 1 | 7 | 0 | 0 | 6 | 0 | 0 | 7 | 0 | 0 | 13 | 0 | 0 | 10 | 0 | 0 |
| 2 | 8 | 0 | 0 | 14 | 0 | 0 | 7 | 0 | 0 | 9 | 1 | 0 | 10 | 0 | 0 |
| 3 | 11 | 0 | 0 | - | - | - | 14 | 0 | 0 | 10 | 0 | 0 | 13 | 0 | 0 |

**Table 6. Results of egg hatching following treatment with 6,6'-Dimethyl-2,2'-dipyridyl at 24 hr intervals post egg laying. The results are indicated for: N (number of eggs per replicate), H ( number of eggs successfully hatched) and Ph (number of eggs partly hatched).**

| Replicates | 24hr | | | 48hr | | | 72hr | | | 96hr | | | 120hr | | |
|---|---|---|---|---|---|---|---|---|---|---|---|---|---|---|---|
| | N | H | Ph | N | H | Ph | N | H | Ph | N | H | Ph | N | H | Ph |
| 1 | 10 | | 0 | 13 | 0 | 0 | 15 | 0 | 0 | 25 | 0 | 0 | 23 | 0 | 0 |
| 2 | 10 | 0 | 0 | 11 | 0 | 0 | 16 | 0 | 0 | 22 | 0 | 0 | 9 | 0 | 0 |
| 3 | 11 | 0 | 0 | 6 | 0 | 0 | 10 | 0 | 0 | 18 | 0 | 0 | - | - | - |

The results from these studies indicate that both 6,6'-Dimethyl-2,2'-dipyridyl and 2,2'-dipyridyl displayed very strong ovicidal activity whereby lice egg hatching was completely inhibited at all time points examined. Both 6,6'-Dimethyl-2,2'-dipyridyl and 2,2'-dipyridyl are metal chelating agents and metalloprotease inhibitors that are non-intercalating.

### Example 10: comparative assessment of commercial lice products with 1,10-phenanthroline

The ovicidal properties of three major commercial head lice products were evaluated in the standard lice egg-hatching assay. The 3 commercial head lice products were as follows:
1. KP-24® Nelson Laboratories, active ingredients 1% maldison (malathion);
2. RID® Bayer, active ingredients, 1% pyrethrins; and
3. NIX® Pfizer, active ingredients, 1% permethrin.

These three products were tested according to manufacturer's recommendations. Groups of eggs (24 hr old) were treated with the different products according to manufacturer's recommendations for the appropriate period of time (5-10 minutes) followed by a rinse for 1-2 minutes in 32°C water. A positive controls (10mM 1,10-phenanthroline) and two negative controls (no treatment and 20% Methanol) were also incorporated. Post exposure to the different products, the eggs were rinsed with warm water at 32°C before being blotted dry on tissue paper and placed at 32°C, 70% RH and left to hatch. A second group of eggs, (48hr old) were treated as previously described and also left to hatch. This process was repeated at 24 hr intervals on head lice eggs up to 120 hr post laying. This method of assaying inhibitors more closely mirrors the field situation where lice eggs will be at various stages of development on the head and permits the inhibitory effects to be observed on these different stages of the parasite. The results of these studies are shown in Table 7.

**Table 7. Results of egg hatching following treatment with 3 commercial head lice products, 10mM 1,10-phenanthroline and controls at 24 hr intervals post egg laying. The results are indicated for: N (number of eggs per replicate), H (number of eggs successfully hatched) and Ph (number of eggs partly hatched).**

| **NIX-Pfizer** | | | | | | | | | | | | | | | |
|---|---|---|---|---|---|---|---|---|---|---|---|---|---|---|---|
| Replicates | 24hr | | | 48hr | | | 72hr | | | 96hr | | | 120hr | | |
| | N | H | Ph | N | H | Ph | N | H | Ph | N | H | Ph | N | H | Ph |
| 1 | 16 | 12 | 2 | 9 | 7 | 0 | 18 | 3 | 3 | 12 | 8 | 3 | 19 | 12 | 3 |
| 2 | 10 | 4 | 3 | 6 | 2 | 3 | 10 | 3 | 3 | 15 | 7 | 5 | 18 | 8 | 7 |
| 3 | 10 | 7 | 2 | 9 | 4 | 3 | 17 | 5 | 7 | - | - | - | 36 | 21 | 5 |

| **RID-Bayer** | | | | | | | | | | | | | | | |
|---|---|---|---|---|---|---|---|---|---|---|---|---|---|---|---|
| Replicates | 24hr | | | 48hr | | | 72hr | | | 96hr | | | 120hr | | |
| | N | H | Ph | N | H | Ph | N | H | Ph | N | H | Ph | N | H | Ph |
| 1 | 8 | 0 | 3 | 12 | 3 | 4 | 7 | 0 | 0 | 8 | 0 | 0 | 14 | 0 | 1 |
| 2 | 8 | 2 | 5 | 7 | 0 | 1 | 5 | 1 | 2 | 8 | 0 | 0 | - | - | - |
| 3 | 5 | 0 | 2 | 10 | 0 | 2 | 6 | 1 | 3 | 11 | 0 | 0 | - | - | - |

| **KP24KP24** | | | | | | | | | | | | | | | |
|---|---|---|---|---|---|---|---|---|---|---|---|---|---|---|---|
| Replicates | 24hr | | | 48hr | | | 72hr | | | 96hr | | | 120hr | | |
| | N | H | Ph | N | H | Ph | N | H | Ph | N | H | Ph | N | H | Ph |
| 1 | 7 | 7 | 0 | 10 | 10 | 0 | 10 | 1 | 3 | 10 | 0 | 0 | 10 | 0 | 0 |
| 2 | 6 | 6 | 0 | 10 | 9 | 0 | 0 | 0 | 0 | 7 | 0 | 0 | 8 | 0 | 0 |
| 3 | 9 | 8 | 0 | - | - | - | - | - | - | - | - | - | 12 | 0 | 1 |

| **1,10-phenanthroline (10mM)** | | | | | | | | | | | | | | | |
|---|---|---|---|---|---|---|---|---|---|---|---|---|---|---|---|
| Replicates | 24hr | | | 48hr | | | 72hr | | | 96hr | | | 120hr | | |
| | N | H | Ph | N | H | Ph | N | H | Ph | N | H | Ph | N | H | Ph |
| 1 | 13 | 0 | 0 | 5 | 0 | 0 | 7 | 0 | 0 | 10 | 0 | 0 | 9 | 0 | 0 |
| 2 | 9 | 0 | 0 | 15 | 0 | 0 | 7 | 0 | 0 | 10 | 0 | 0 | 6 | 4 | 0 |
| 3 | - | - | - | 8 | 0 | 0 | 9 | 0 | 0 | - | - | - | 7 | 1 | 0 |

| **Control (20% Methanol)** | | | | | | | | | | | | | | | |
|---|---|---|---|---|---|---|---|---|---|---|---|---|---|---|---|
| Replicates | 24hr | | | 48hr | | | 72hr | | | 96hr | | | 120hr | | |
| | N | H | Ph | N | H | P | N | H | P | N | H | P | N | H | Ph |
| 1 | - | - | - | 14 | 14 | 0 | 10 | 10 | 0 | 10 | 10 | 0 | 13 | 13 | 0 |
| 2 | - | - | - | 5 | 4 | 0 | 8 | 8 | 0 | 10 | 9 | 0 | 7 | 7 | 0 |
| 3 | - | - | - | - | - | 0 | 9 | 7 | 0 | 4 | 4 | 0 | 10 | 10 | 0 |

| **Control (Untreated)** | | | | | | | | | | | | | | | |
|---|---|---|---|---|---|---|---|---|---|---|---|---|---|---|---|
| Replicates | 24hr | | | 48hr | | | 72hr | | | 96hr | | | 120hr | | |
| | N | H | Ph | N | H | P | N | H | P | N | H | P | N | H | Ph |
| 1 | 10 | 9 | 0 | 11 | 11 | 0 | 25 | 24 | 0 | 10 | 8 | 0 | 20 | 20 | 0 |
| 2 | 20 | 18 | 0 | 8 | 7 | 0 | 10 | 10 | 0 | 11 | 10 | 0 | 20 | 18 | 0 |
| 3 | - | - | - | 8 | 8 | 0 | - | - | - | 10 | 10 | 0 | - | - | - |

Results from the testing of 3 commercial pediculicides indicate that they displayed inconsistent levels of ovicidal activity across the different stages of lice egg hatching. Whereas, the compound 1,10-phenanthroline was highly effective at inhibiting lice egg hatching.

### Example 11: Assessment of additional commercial lice products

The ovicidal properties of two major commercial head lice products were evaluated in the standard lice egg-hatching assay. The 2 commercial head lice products were as follows:
1. Pronto Plus® Shampoo Del Laboratories, active ingredients 0.33% Pyrethrins; and
2. Pronto Plus® Mousse Shampoo Del Laboratories, active ingredients, 0.33% Pyrethrins.

These two products were tested according to manufacturer's recommendations. Groups of eggs (24 hr old) were treated with the different products according to manufacturer's recommendations for the appropriate period of time (5-10 minutes) followed by a rinse for 1-2 minutes in 32°C water. Two negative controls (no treatment and 20% ethanol) were also incorporated. Post exposure to the different products, the eggs were blotted dry on tissue paper and placed at 32°C, 70% RH and left to hatch. A second group of eggs, (48hr old) were treated as previously described and also left to hatch. This process was repeated at 24 hr intervals on head lice eggs up to 120 hr post laying. This method of assaying inhibitors more closely mirrors the field situation where lice eggs will be at various stages of development on the head and permits the inhibitory effects to be observed on these different stages of the parasite. The results of these studies are shown in Table 8.

**Table 8. Results of egg hatching following treatment with 2 commercial head lice products and controls at 24 hr intervals post egg laying. The results are indicated for: N (number of eggs per replicate), H (number of eggs successfully hatched) and Ph (number of eggs partly hatched).**

| **Pronto Plus Shampoo** | | | | | | | | | | | | | | | |
|---|---|---|---|---|---|---|---|---|---|---|---|---|---|---|---|
| Replicates | 24hr | | | 48hr | | | 72hr | | | 96hr | | | 120hr | | |
| | N | H | Ph | N | H | P | N | H | P | N | H | P | N | H | Ph |
| 1 | 14 | 10 | 2 | 11 | 9 | 0 | 30 | 27 | 0 | 35 | 30 | 0 | 40 | 38 | 2 |
| 2 | 20 | 15 | 3 | 21 | 18 | 0 | 19 | 16 | 0 | 42 | 36 | 0 | 38 | 29 | 5 |
| 3 | - | - | - | - | - | - | - | - | - | - | - | - | - | - | - |

| **Pronto Plus Mousse Shampoo** | | | | | | | | | | | | | | | |
|---|---|---|---|---|---|---|---|---|---|---|---|---|---|---|---|
| Replicates | 24hr | | | 48hr | | | 72hr | | | 96hr | | | 120hr | | |
| | N | H | Ph | N | H | P | N | H | Ph | N | H | Ph | N | H | Ph |
| 1 | 10 | 8 | 0 | 18 | 15 | 0 | 47 | 31 | 9 | 63 | 8 | 34 | 51 | 7 | 40 |
| 2 | 15 | 13 | 0 | 10 | 6 | 0 | 30 | 14 | 8 | 29 | 5 | 10 | 50 | 8 | 30 |
| 3 | 11 | 9 | 0 | - | - | - | 34 | 13 | 17 | 21 | 1 | 15 | 31 | 1 | 17 |

| **Control (ethanol)** | | | | | | | | | | | | | | | |
|---|---|---|---|---|---|---|---|---|---|---|---|---|---|---|---|
| Replicates | 24hr | | | 48hr | | | 72hr | | | 96hr | | | 120hr | | |
| | N | H | Ph | N | H | P | N | H | P | N | H | P | N | H | P |
| 1 | 12 | 10 | 0 | 18 | 16 | 0 | 40 | 36 | 1 | 21 | 20 | 0 | 49 | 47 | 0 |
| 2 | 11 | 9 | 0 | 21 | 18 | 0 | 41 | 37 | 0 | 28 | 26 | 0 | 39 | 36 | 0 |
| 3 | 11 | 11 | 0 | 13 | 11 | 0 | 75 | 70 | 0 | 29 | 27 | 0 | 36 | 34 | 0 |

| **Control (untreated)** | | | | | | | | | | | | | | | |
|---|---|---|---|---|---|---|---|---|---|---|---|---|---|---|---|
| Replicates | 24hr | | | 48hr | | | 72hr | | | 96hr | | | 120hr | | |
| | N | H | Ph | N | H | P | N | H | P | N | H | P | N | H | P |
| 1 | 10 | 9 | 0 | 27 | 26 | 0 | 61 | 60 | 0 | 50 | 49 | 1 | 48 | 46 | 0 |
| 2 | - | - | - | - | - | - | - | - | - | - | - | - | - | - | - |
| 3 | - | - | - | - | - | - | - | - | - | - | - | - | - | - | - |

Results from the testing of 2 commercial pediculicides indicate that they displayed very poor and inconsistent ovicidal activity across the different stages of lice egg hatching.

### References:

Al-Sayah, M.H., McDonald, R., Branda, N.R., Euro. J. Org. Chem., 2004, 173-182.
Buhleier, E., Wehner, W., Vögthe, F., Chem. Ber., 1978,111, 200-204.
Busvine, J.R.,. Entomology and evolution. Antenna. 1993, 17: 196-201.
Busvine, J.R, Biology of the parasites. Cutaneous Infestations and Insect Bites (M. Orkin and H.I. Maibach, eds).1985, pp.163-174. New York: Marcel Dekker.
Dymock, J.J., Laing W.A., Shaw B.D., Gatehouse A.M.R, Cristellar. J.T. New Zealand Journal of Zoology, 1992, 19: 123-131.
Green, T.W. and Wutz, P., Protecting groups of organic synthesis, John Wiley & Son, 3rd Edition, 1999.
Imperiali, B. and Fisher, S.L., J. Org. Chem., 1992, 57, 757-759.
Jones, J., Amino acid synthesis and peptide synthesis, Oxford Chemistry Press, 1992.
Samuels R. I. and Paterson J. C. Comparative Biochemistry and Physiology. 1995 110B: 661-669.

## Claims

1. Use of at least one metal chelating agent or a pharmaceutically, veterinary or agriculturally acceptable salt thereof, for the manufacture of a topical composition for use in inhibiting hatching of an ectoparasite egg on a human or animal host, wherein the at least one metal chelating agent is a compound of formula (Ia): 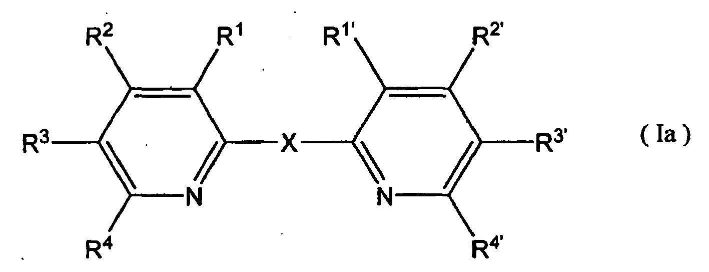
wherein X is a covalent bond, CH₂-Z-CH₂ or -Z-
wherein R¹ and R^{1'} are independently selected from hydrogen, or C₁₋₃alkyl
wherein at least one of R², R^{2'}, R³, R^{3'}, R⁴ and R^{4'} is a methyl group and the remaining R², R^{2'}, R³, R^{3'}, R⁴ and R^{4'} are independently selected from hydrogen or C₁₋₃ alkyl; and
Z is selected from a covalent bond, -NH-, **-**O-**,** -S-, -C(O)- and -C(S)-.

2. A method for inhibiting hatching of an ectoparasite egg, excluding use on a human or animal host, comprising exposing the ectoparasite egg to a topical composition comprising at least one metal chelating agent, wherein the at least one metal chelating agent is a compound of formula (Ia): 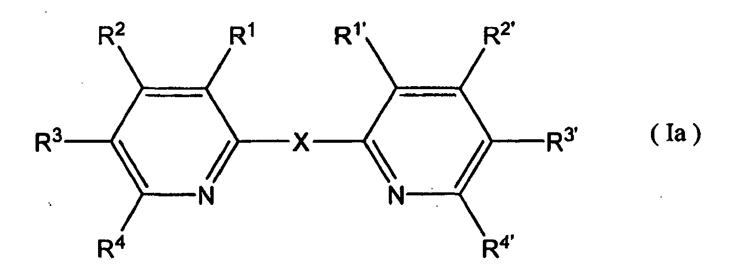
wherein X is a covalent bond, CH₂-Z-CH₂ or -Z-
wherein R¹ and R^{1'} are independently selected from hydrogen, or C₁₋₃alkyl
wherein at least one of R², R², R³, R^{3'}, R⁴ and R^{4'} is a methyl group and the remaining R², R^{2'}, R³, R^{3'}, R⁴ and R^{4'} are independently selected from hydrogen or C₁₋₃ alkyl; and
Z is selected from a covalent bond, -NH-, **-**O**-,** -S-, -C(O)- and -C(S)-;
or a pharmaceutically, veterinary or agriculturally acceptable salt thereof.

3. Use of an effective amount of at least one metal chelating agent or a pharmaceutically, veterinary or agriculturally acceptable salt thereof, for the manufacture of a topical medicament for use in treating ectoparasite infestation in a human or animal host, wherein the at least one metal chelating agent is a compound of formula (Ia): 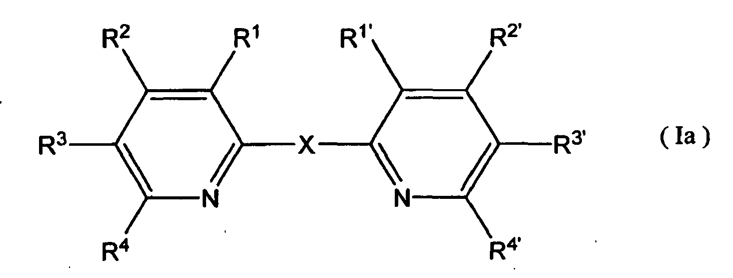
wherein X is a covalent bond, CH₂-Z-CH₂ or -Z-
wherein R¹ and R^{1'} are independently selected from hydrogen, or C₁₋₃alkyl
wherein at least one of R², R^{2'}, R³, R^{3'}, R⁴ and R^{4'} is a methyl group and the remaining R², R^{2'}, R³, R^{3'} R⁴ and R^{4'} are independently selected from hydrogen or C₁₋₃ alkyl; and
Z is selected from a covalent bond, -NH-, -O-, -S-, -C(O)- and -C(S)-.

4. A method of treating ectoparasite infestation, excluding use on a human or animal host, comprising exposing the host to an effective amount of at least one metal chelating agent, wherein the at least one metal chelating agent is a compound of formula (Ia): 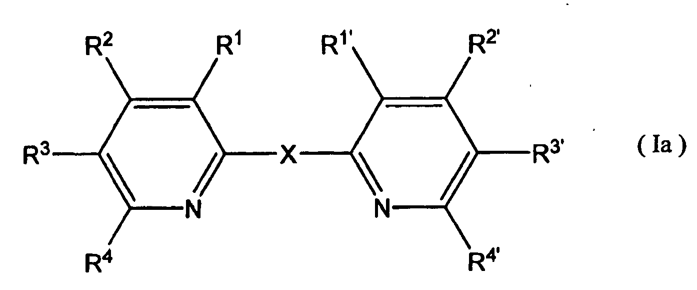
wherein X is a covalent bond, CH₂-Z-CH₂ or -Z-
wherein R¹ and R^{1'} are independently selected from hydrogen, or C₁₋₃alkyl
wherein at least one of R², R^{2'}, R³, R^{3'}, R⁴ and R^{4'} is a methyl group and the remaining R², R^{2'}, R³, R^{3'}, R⁴ and R^{4'} are independently selected from hydrogen or C₁₋₃ alkyl; and
Z is selected from a covalent bond, -NH-, -O-, -S-, -C(O)- and -C(S)-;
or a pharmaceutically, veterinary or agriculturally acceptable salt thereof.

5. The use or method according to claim 2 or claim 4, wherein the ectoparasite egg is present on: host plants including cereal crops, fruit trees, cotton, oil seed crops, ornamental plants, flowers, vine crops, root crops, pasture plants and vegetables, or on breeding sites such as: houses and buildings, enclosures for domestic and farming animals, carpets, blankets, curtains and furniture.

6. The use or method according to any preceding claim, wherein Z is -NH-, -O- or S.

7. The use or method according to any preceding claim, wherein the compound of formula (Ia) is selected from:
6,6'-dimethyl-2,2'-dipyridyl,
5,5'-dimethyl-2,2'-dipyridyl, .
4,4'-dimethyl-2,2'-dipyridyl,
or a pharmaceutically, veterinary or agriculturally acceptable salt thereof.

8. The use or method according to any preceding claim, wherein the ectoparasite egg is laid by an ectoparasite of a species from an order selected from the group consisting of Lepidoptera, Hemiptera, Orthoptera, Psocoptera, Hymenoptera, Isoptera, Coleoptera, Dictyoptera, Thysanoptera, Homoptera, Diptera, Anaplura, Malophaga, Siphonaptera, Arachnida and Phthiraptera.

9. The use or method according to any preceding claim, wherein the ectoparasite egg is laid by an ectoparasite of a species selected from the group consisting of Helicoverpa spp. Crocidolomia pavonana (Cabbage cluster caterpillar), Pieris rapae (Cabbage white butterfly), Phthorimaea operculella (Potatoe moth), Chrsyodexis spp. (Tobacco loopers), Plutella xylostella (Diamondback moth), Eiphyas postvittana (Walker)(light briown apple moth), Bovicola ovis (Sheep louse), Bovicola bovis, Haemotopinus eurystemus (short-nosed cattle louse), Linognathus vituli (long nosed cattle louse), Solenoptes scabiei suis, Sarcoptes scabiei bovis, Psoroptes ovis, Pthirus pubis, Pediculus humanus capitis, Pedicululs humanus humanus, Sarcoptes scabiei var, humani and Dermatophgoides spp.

10. The use or method according to any preceding claim, wherein the ectoparasite infestation is a lice infestation.

11. The use or method according to any preceding claim, wherein the metal chelating agent is applied simultaneously, separately or sequentially with a second ectoparasiticide.

12. The use or method according to claim 11, wherein the second ectoparasiticide controls nymphs and/or adult ectoparasites.

13. The use or method according to any preceding claim, wherein said composition is formulated for use in a direct topical application in a form selected from the group consisting of a dip, spray, aerosol, shampoo, mousse, emulsion, solution, cream, dust, lotion, foams, microemulsions and jellies.

14. The use or method according to claim 1 or claim 3" wherein said ectoparasite egg is human head lice eggs and the composition is formulated for use in a topical application to human scalp and is in a form selected from the group consisting of a dip, spray, aerosol, shampoo, mousse, emulsion, solution, cream, dust, lotion, foams, microemulsions and jellies.

15. The use or method according to any preceding claim, wherein said composition includes: water, salt solutions, alcohols, polyethylene glycols, gelatine, lactose, magnesium sterate, or silicic acid.

16. At least one metal chelating agent or a pharmaceutically, veterinary or agriculturally acceptable salt thereof, for use in inhibiting hatching of an ectoparasite egg on a human or animal host, wherein the at least one metal chelating agent is a compound of formula (Ia): 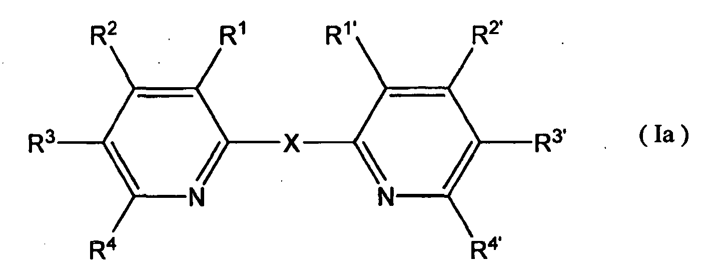
wherein X is a covalent bond, CH₂-Z-CH₂ or -Z-
wherein R¹ and R^{1'} are independently selected from hydrogen, or C₁₋₃alkyl
wherein at least one of R², R^{2'}, R³, R^{3'}, R⁴ and R^{4'} is a methyl group and the remaining R², R^{2'}, R³, R^{3'}, R⁴ and R^{4'} are independently selected from hydrogen or C₁₋₃ alkyl; and
Z is selected from a covalent bond, -NH-, -O-, -S-, -C(O)- and -C(S)-.

17. A method of inhibiting hatching of an ectoparasite egg, excluding use on a human or animal host, comprising exposing the ectoparasite egg to at least one metal chelating agent or a pharmaceutically, veterinary or agriculturally acceptable salt thereof, wherein the at least one metal chelating agent is a compound of formula (Ia): 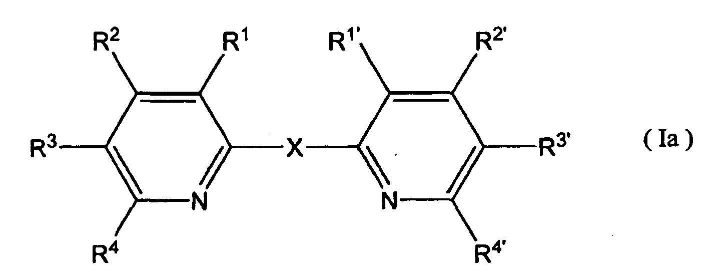
wherein X is a covalent bond, CH₂-Z-CH₂ or -Z-
wherein R¹ and R^{1'} are independently selected from hydrogen, or C₁₋₃alkyl
wherein at least one of R², R^{2'}, R³, R^{3'}, R⁴ and R^{4'} is a methyl group and the remaining R², R^{2'}, R³, R^{3'}, R⁴ and R^{4'} are independently selected from hydrogen or C₁₋₃ alkyl; and
Z is selected from a covalent bond, -NH-, -O-, -S-, -C(O)- and -C(S)-.

18. The method according to claim 17, wherein the ectoparasite egg is present on: host plants including cereal crops, fruit trees, cotton, oil seed crops, ornamental plants, flowers, vine crops, root crops, pasture plants and vegetables, or on breeding sites such as: houses and buildings, enclosures for domestic and farming animals, carpets, blankets, curtains and furniture.

19. The use, method or agent according to any preceding claim, wherein the compound of formula (Ia) is 5,5' dimethyl-2-2'dipyridyl, or a pharmaceutically, veterinary or agriculturally acceptable salt thereof.

## Patentansprüche

1. Verwendung zumindest eines Metallchelatbildners oder eines pharmazeutisch, veterinär oder landwirtschaftlich annehmbaren Salzes davon für die Herstellung einer topischen Zusammensetzung für die Verwendung beim Verhindern des Ausbrütens eines Ektoparasiteneies bei bzw. auf einem Menschen- oder Tierwirt, wobei der zumindest eine Metallchelatbildner eine Zusammensetzung der Formel (Ia) 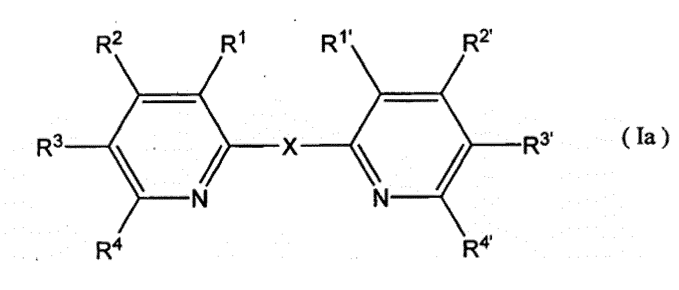 ist,
wobei X eine Atombindung CH₂-Z-CH₂ oder -Z- ist,
wobei R¹ und R^{1'} unabhängig aus Wasserstoff oder C₁₋₃Alkyl ausgewählt sind,
wobei zumindest eines von R², R^{2'}, R³, R^{3'}, R⁴ und R^{4'} eine Methylgruppe ist,
wobei die übrigen R², R^{2'}, R³, R^{3'}, R⁴ und R^{4'} unabhängig aus Wasserstoff oder C₁₃Alkyl ausgewählt sind
und wobei Z aus einer Atombindung -NH-, -O-, -S-, -C(O)- und -C(S)- ausgewählt ist.

2. Verfahren zum Verhindern des Ausbrütens eines Ektoparasiteneies, ausnehmend die Anwendung bei einem Menschen- oder Tierwirt, umfassend das Aussetzen des Ektoparasiteneies einer topischen Zusammensetzung, umfassend zumindest einen Metallchelatbildner, wobei der zumindest eine Metallchelatbildner eine Zusammensetzung der Formel (Ia) 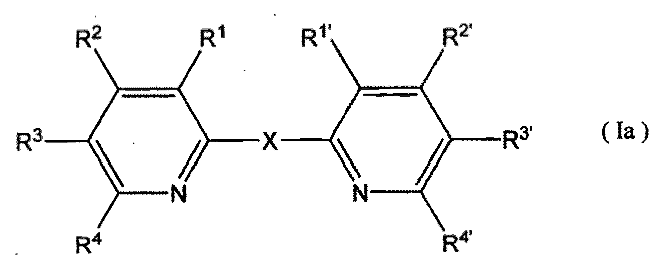 ist,
wobei X eine Atombindung CH₂-Z-CH₂ oder -Z- ist,
wobei R¹ und R^{1'} unabhängig aus Wasserstoff oder C₁₋₃Alkyl ausgewählt sind,
wobei zumindest eines von R², R^{2'}, R³, R^{3'}, R⁴ und R^{4'} eine Methylgruppe ist,
wobei die übrigen R², R^{2'}, R³, R^{3'}, R⁴ und R^{4'} unabhängig aus Wasserstoff oder C₁₃Alkyl ausgewählt sind
und wobei Z aus einer Atombindung -NH-, -O-, -S-, -C(O)- und -C(S)- ausgewählt ist oder ein pharmazeutisch, veterinär oder landwirtschaftlich annehmbares Salz davon ist.

3. Verwendung einer wirksamen Menge zumindest eines Metallchelatbildners oder eines pharmazeutisch, veterinär oder landwirtschaftlich annehmbaren Salzes davon zur Herstellung eines topischen Medikaments für die Verwendung bei der Behandlung eines Ektoparasitenbefalls eines Menschen- oder Tierwirts, wobei der zumindest eine Metallchelatbildner eine Zusammensetzung der Formel (Ia) 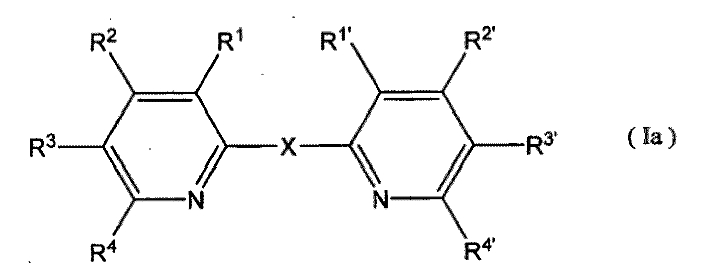 ist,
wobei X eine Atombindung CH₂-Z-CH₂ oder -Z- ist,
wobei R¹ und R^{1'} unabhängig aus Wasserstoff oder C₁₋₃Alkyl ausgewählt sind,
wobei zumindest eines von R², R^{2'}, R³, R^{3'}, R⁴ und R^{4'} eine Methylgruppe ist,
wobei die übrigen R², R^{2'}, R³, R^{3'}, R⁴ und R^{4'} unabhängig aus Wasserstoff oder C₁₃Alkyl ausgewählt sind
und wobei Z aus einer Atombindung -NH-, -O-, -S-, -C(O)- und -C(S)- ausgewählt ist.

4. Verfahren zum Behandeln eines Ektoparasitenbefalls, ausnehmend die Anwendung bei einem Menschen- oder Tierwirt, umfassend das Aussetzen des Wirtes einer wirksamen Menge zumindest eines Metallchelatbildners, wobei der zumindest eine Metallchelatbildner eine Zusammensetzung der Formel (Ia) 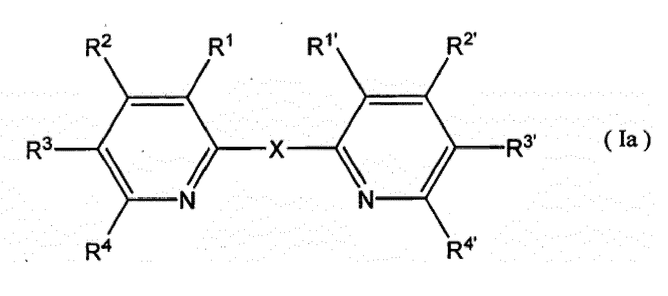 ist,
wobei X eine Atombindung CH₂-Z-CH₂ oder -Z- ist,
wobei R¹ und R^{1'} unabhängig aus Wasserstoff oder C₁₋₃Alkyl ausgewählt sind,
wobei zumindest eines von R², R^{2'}, R³, R^{3'}, R⁴ und R^{4'} eine Methylgruppe ist,
wobei die übrigen R², R^{2'}, R³, R^{3'}, R⁴ und R^{4'} unabhängig aus Wasserstoff oder C₁₃Alkyl ausgewählt sind
und wobei Z aus einer Atombindung -NH-, -O-, -S-, -C(O)- und -C(S)- ausgewählt ist oder ein pharmazeutisch, veterinär oder landwirtschaftlich annehmbares Salz davon ist.

5. Verwendung oder Verfahren nach Anspruch 2 oder 4, wobei das Ektoparasitenei auf Wirtspflanzen, enthaltend Getreidefrüchte, Obstbäume, Baumwolle, Ölsamenfrüchte, Zierpflanzen, Blumen, Weinstockpflanzen, Wurzelpflanzen, Futterpflanzen und Gemüse, oder in Brutstellen, wie Häusern und Gebäuden, Gehegen für Haustiere und Landwirtschaftstiere, Teppichen, Bettdecken, Vorhängen und Möbel vorhanden ist.

6. Verwendung oder Verfahren nach irgendeinem vorhergehenden Anspruch, wobei Z gegeben ist durch -NH-, -O- oder S.

7. Verwendung oder Verfahren nach irgendeinem vorhergehenden Anspruch, wobei die Zusammensetzung der Formel (Ia) ausgewählt wird aus
6,6'-Dimethyl-2,2'-Dipyridyl,
5,5'-Dimethyl-2,2'-Dipyridyl,
4,4'-Dimethyl-2,2'-Dipyridyl
oder einem pharmazeutisch oder veterinär oder landwirtschaftlich annehmbaren Salz davon.

8. Verwendung oder Verfahren nach irgendeinem vorhergehenden Anspruch, wobei das Ektoparasitenei von einem Ektoparasiten einer Spezies aus einer Ordnung abgelegt ist, die aus der Gruppe ausgewählt ist, bestehend aus Lepidoptera, Hemiptera, Orthoptera, Psocoptera, Hymenoptera, Isoptera, Coleoptera, Dictyoptera, Thysanoptera, Homoptera, Diptera, Anaplura, Malophaga, Siphonaptera, Arachnida und Phthiraptera.

9. Verwendung oder Verfahren nach irgendeinem vorhergehenden Anspruch, wobei das Ektoparasitenei von einem Parasiten einer Spezies abgelegt ist, die aus der Gruppe ausgewählt ist, bestehend aus Helicoverpa spp. Crocidolomia pavonana (Kohlclusterraupe), Pieris rapae (Kohlweißling), Phthorimaea operculella (Kartoffelmotte), Chrsyodexis spp. (Tabakspanner bzw. -raupen), Plutella xylostella (Diamantschwarzmotte), Eiphyas postvittana (Walker) (hellbraune Apfelmotte), Bovicola ovis (Schaflaus), Bovicola bovis, Haemotopinus eurystemus (kurznasige Rinderlaus), Linognathus vituli (langnasige Rinderlaus), Solenoptes scabiei suis, Sarcoptes scabiei bovis, Psoroptes ovis, Pthirus pubis, Pediculus humanus capitis, Pediculus humanus humanus, Sarcoptes scabiei var, humani und Dermatophgoides spp.

10. Verwendung oder Verfahren nach irgendeinem vorhergehenden Anspruch, wobei der Ektoparasitenbefall ein Läusebefall ist.

11. Verwendung oder Verfahren nach irgendeinem vorhergehenden Anspruch, wobei der Metallchelatbildner gleichzeitig, separat oder aufeinanderfolgend mit einem zweiten Ektoparasitizid angewandt wird.

12. Verwendung oder Verfahren nach Anspruch 11, wobei das zweite Ektoparasitizid Puppen- und/oder adulte Ektoparasiten unter Kontrolle hat.

13. Verwendung oder Verfahren nach irgendeinem vorhergehenden Anspruch, wobei die Zusammensetzung festgelegt wird für die Verwendung in einer direkten topischen Anwendung in einer Form, die aus der Gruppe ausgewählt ist, bestehend aus einer Tauchflüssigkeit, einem Spray, einem Aerosol, einem Shampoo, einer Mousse, einer Emulsion, einer Lösung, einer Creme, einem Puder, einer Lotion, Schäumen, Mikroemulsionen und Gelees.

14. Verwendung oder Verfahren nach Anspruch 1 oder 3, wobei das Ektoparasitenei ein Läuseei des Menschenkopfes ist und wobei die Zusammensetzung für die Verwendung in einer topischen Anwendung auf der Kopfhaut eines Menschen festgelegt wird und in einer Form ist, die aus der Truppe ausgewählt ist, bestehend aus einer Tauchflüssigkeit, einem Spray, einem Aerosol, einem Shampoo, einer Mousse, einer Emulsion, einer Lösung, einer Creme, einem Puder, einer Lotion, Schäumen, Mikroemulsionen und Gelees.

15. Verwendung oder Verfahren nach irgendeinem vorhergehenden Anspruch, wobei die Zusammensetzung Wasser, Salzlösungen, Alkohole, Polyethylenglykole, Gelatine, Laktose, Magnesiumstearat oder Kieselsäure enthält.

16. Zumindest ein Metallchelatbildner oder ein pharmazeutisch, veterinär oder landwirtschaftlich annehmbares Salz davon zur Verwendung beim Verhindern des Ausbrütens eines Ektoparasiteneies auf einem Menschen- oder Tierwirt,
wobei der zumindest eine Metallchelatbildner eine Zusammensetzung der Formel (Ia) 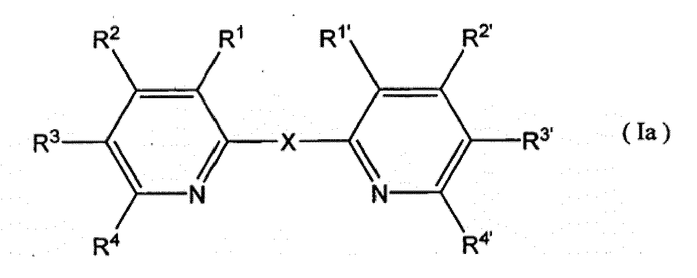 ist,
wobei X eine Atombindung CH₂-Z-CH₂ oder -Z- ist,
wobei R¹ und R^{1'} unabhängig aus Wasserstoff oder C₁₋₃Alkyl ausgewählt sind,
wobei zumindest eines von R², R^{2'}, R³, R^{3'}, R⁴ und R^{4'} eine Methylgruppe ist,
wobei die übrigen R², R^{2'}, R³, R^{3'}, R⁴ und R^{4'} unabhängig aus Wasserstoff oder C₁₃Alkyl ausgewählt sind
und wobei Z aus einer Atombindung -NH-, -O-, -S-, -C(O)- und -C(S)- ausgewählt ist.

17. Verfahren zum Verhindern des Ausbrütens eines Ektoparasiteneies, ausnehmend die Verwendung bei einem Menschen- oder Tierwirt, umfassend das Aussetzen des Ektoparasiteneies zumindest einem Metallchelatbildner oder einem pharmazeutisch, veterinär oder landwirtschaftlich annehmbaren Salz davon,
wobei der zumindest eine Metallchelatbildner eine Zusammensetzung der Formel (Ia) 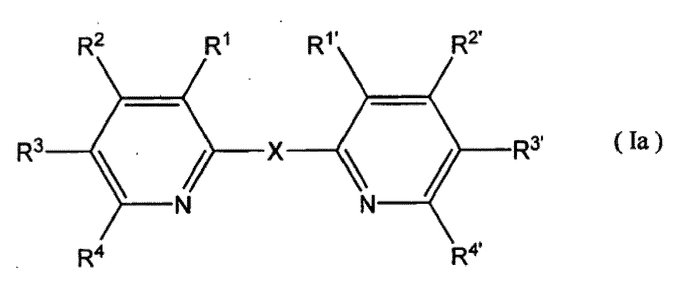 ist,
wobei X eine Atombindung CH₂-Z-CH₂ oder -Z- ist,
wobei R¹ und R^{1'} unabhängig aus Wasserstoff oder C₁₋₃Alkyl ausgewählt sind,
wobei zumindest eines von R², R^{2'}, R³, R^{3'}, R⁴ und R^{4'} eine Methylgruppe ist,
wobei die übrigen R² R^{2'}, R³, R^{3'}, R⁴ und R^{4'} unabhängig aus Wasserstoff oder C₁₃Alkyl ausgewählt sind
und wobei Z aus einer Atombindung -NH-, -O-, -S-, -C(O)- und -C(S)- ausgewählt ist.

18. Verfahren nach Anspruch 17, wobei das Ektoparasitenei auf Wirtspflanzen, enthaltend Getreidefrüchte, Obstbäume, Baumwolle, Ölsamenfrüchte, Zierpflanzen, Blumen, Weinstockpflanzen, Wurzelpflanzen, Futterpflanzen und Gemüse, oder in Brutstellen, wie Häusern und Gebäuden, Gehegen für Haustiere und Landwirtschaftstiere, Teppichen, Bettdecken, Vorhängen und Möbel vorhanden ist.

19. Verwendung, Verfahren oder Mittel nach irgendeinem vorhergehenden Anspruch, wobei die Zusammensetzung der Formel (Ia) 5,5'Dimethyl-2-2'Dipyridyl oder ein pharmazeutisch, veterinär oder landwirtschaftlich annehmbares Salz davon ist.

## Revendications

1. Utilisation d'au moins un agent chélateur métallique ou d'un sel de celui-ci acceptable sur le plan pharmaceutique, vétérinaire ou agricole pour la fabrication d'une composition topique destinée à empêcher l'éclosion d'un oeuf d'ectoparasite sur un hôte humain ou animal, dans laquelle ledit au moins un agent chélateur métallique est un composé de formule (la) : 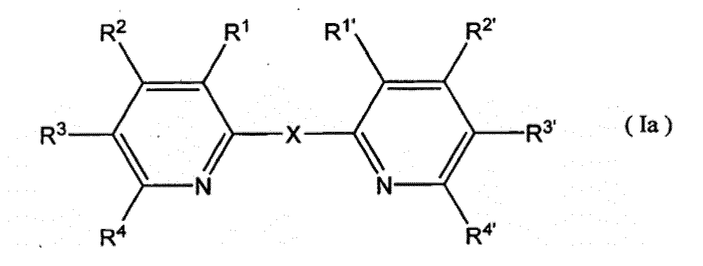
dans laquelle X est une liaison covalente CH₁-Z-CH₁ ou -Z-
dans laquelle R¹ et R^{1'} sont sélectionnés indépendamment parmi hydrogène ou C₁₋₃ alkyle
dans laquelle au moins un des R², R^{2'}, R³, R^{3'}, R⁴ et R^{4'} est un groupe méthyle et les R², R^{2'}, R³, R^{3'}, R⁴ et R^{4'} restants sont sélectionnés indépendamment parmi hydrogène ou C₁₋₃ alkyle ; et
Z est sélectionné parmi une liaison covalente -NH-, -O-, -S-, -C(O)- et -C(S)-.

2. Procédé pour empêcher l'éclosion d'un oeuf d'ectoparasite, excluant l'utilisation sur un hôte humain ou animal, consistant à exposer l'oeuf d'ectoparasite à une composition topique comprenant au moins un agent chélateur métallique, dans lequel ledit au moins un agent chélateur métallique est un composé de formule (la) : 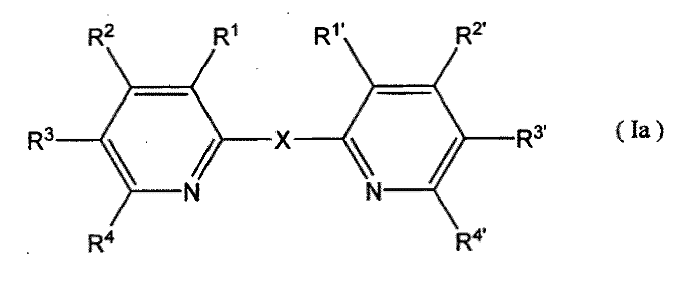
dans laquelle X est une liaison covalente CH₂-Z-CH₂ ou -Z-
dans laquelle R¹ et R^{1'} sont sélectionnés indépendamment parmi hydrogène ou C₁₋₃ alkyle
dans laquelle au moins un des R², R^{2'}, R³, R^{3'}, R⁴ et R^{4'} est un groupe méthyle et les R², R^{2'}, R³, R^{3'}, R⁴ et R^{4'} restants sont sélectionnés indépendamment parmi hydrogène ou C₁₋₃ alkyle ; et
Z est sélectionné parmi une liaison covalente -NH-, -O-, -S-, -C(O)- et -C(S)- ;
ou un sel de celui-ci acceptable sur le plan pharmaceutique, vétérinaire ou agricole.

3. Utilisation d'une quantité efficace d'au moins un agent chélateur métallique ou d'un sel de celui-ci acceptable sur le plan pharmaceutique, vétérinaire ou agricole pour la fabrication d'un médicament topique destiné à traiter l'infestation ectoparasitaire dans un hôte humain ou animal, dans laquelle ledit au moins un agent chélateur métallique est un composé de formule (la) : 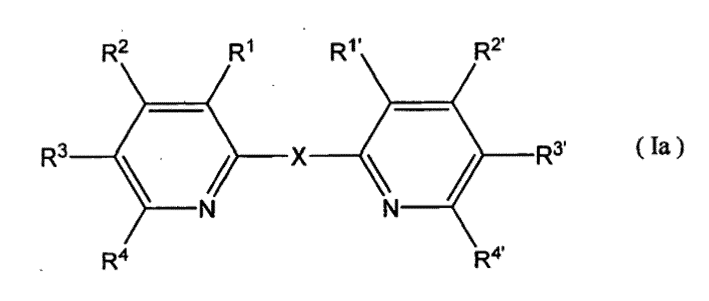
dans laquelle X est une liaison covalente CH₂-Z-CH₂ ou -Z-
dans laquelle R¹ et R^{1'} sont sélectionnés indépendamment parmi hydrogène ou C₁₋₃ alkyle
dans laquelle au moins un des R², R^{2'}, R³, R^{3'}, R⁴ et R^{4'} est un groupe méthyle et les R², R^{2'}, R³, R^{3'}, R⁴ et R^{4'} restants sont sélectionnés indépendamment parmi hydrogène ou C₁₋₃ alkyle ; et
Z est sélectionné parmi une liaison covalente -NH-, -O-, -S-, -C(O)- et -C(S)-.

4. Procédé pour traiter l'infestation ectoparasitaire, excluant l'utilisation sur un hôte humain ou animal, consistant à exposer l'hôte à une quantité efficace d'au moins un agent chélateur métallique, dans lequel ledit au moins un agent chélateur métallique est un composé de formule (la) : 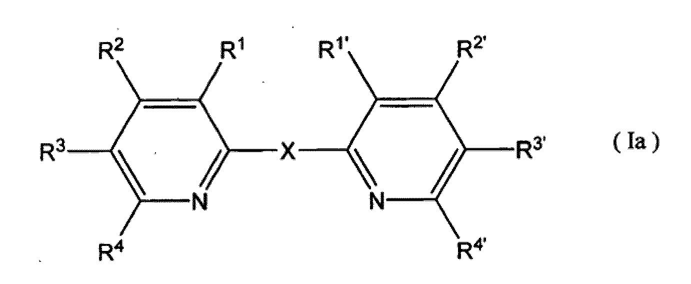
dans laquelle X est une liaison covalente CH₂-Z-CH₂ ou -Z-
dans laquelle R¹ et R^{1'} sont sélectionnés indépendamment parmi hydrogène ou C₁₋₃ alkyle
dans laquelle au moins un des R², R^{2'}, R³, R^{3'}, R⁴ et R^{4'} est un groupe méthyle et les R², R^{2'}, R³, R^{3'}, R⁴ et R^{4'} restants sont sélectionnés indépendamment parmi hydrogène ou C₁₋₃ alkyle ; et
Z est sélectionné parmi une liaison covalente -NH-, -O-, -S-, -C(O)- et -C(S)- ;
ou un sel de celui-ci acceptable sur le plan pharmaceutique, vétérinaire ou agricole.

5. Utilisation ou procédé selon la revendication 2 ou la revendication 4, dans laquelle ou lequel l'oeuf d'ectoparasite est présent sur des plantes hôtes comprenant : cultures céréalières, arbres fruitiers, coton, cultures de graines oléagineuses, plantes ornementales, fleurs, vignes, cultures racines, plantes fourragères et légumes, ou sur des sites de reproductions tels que maisons et bâtiments, enclos pour animaux domestiques et d'élevage, moquettes, couvertures, rideaux et meubles.

6. Utilisation ou procédé selon une quelconque des revendications précédentes, dans laquelle ou lequel Z est -NH-, -O- ou S.

7. Utilisation ou procédé selon une quelconque des revendications précédentes, dans laquelle ou lequel le composé de la formule (Ia) est sélectionné parmi :
6,6'-diméthyle-2,2'-dipyridyle,
5,5'-diméthyle-2,2'-dipyridyle,
4,4'-diméthyle-2,2'-dipyridyle,
ou un sel de ceux-ci acceptable sur le plan pharmaceutique, vétérinaire ou agricole.

8. Utilisation ou procédé selon une quelconque des revendications précédentes, dans laquelle ou lequel l'oeuf d'ectoparasite est pondu par un ectoparasite d'une espèce appartenant à un ordre sélectionné dans le groupe constitué par : lépidoptères, hémiptères, orthoptères, psocoptères, hyménoptères, isoptères, coléoptères, dictyoptères, thysanoptères, homoptères, diptères, anoploures, malophages, siphonaptères, arachnides et phthiraptères.

9. Utilisation ou procédé selon une quelconque des revendications précédentes, dans laquelle ou lequel l'oeuf d'ectoparasite est pondu par un ectoparasite d'une espèce sélectionnée dans le groupe constitué par : Helicoverpa spp. Crocidolomia pavonana (chenille défoliatrice du chou), Pieris rapae (piéride du chou), Phthorimaea operculella (mite de la pomme de terre), Chrsyodexis spp. (fausse-arpenteuse du tabac), Plutella xylostella (fausse teigne des crucifère), Eiphyas postvittana (Walker) (pyrale brun pâle de la pomme), Bovicola ovis (pou du mouton), Bovicola bovis, Haemotopinus eurysternus (pou des bovins à nez court), Linognathus vituli (pou des bovins à long nez), Solenoptes scabiei suis, Sarcoptes scabiei bovis, Psoroptes ovis, Pthirus pubis, Pediculus humanus capitis, Pediculus humanus humanus, Sarcoptes scabiei var, humani et Dermatophgoides spp.

10. Utilisation ou procédé selon une quelconque des revendications précédentes, dans laquelle ou lequel l'infestation ectoparasitaire est une infestation par des poux.

11. Utilisation ou procédé selon une quelconque des revendications précédentes, dans laquelle ou lequel l'agent chélateur métallique est appliqué simultanément, séparément ou séquentiellement avec un deuxième ectoparasiticide.

12. Utilisation ou procédé selon la revendication 11, dans laquelle ou lequel le deuxième ectoparasiticide contrôle des nymphes et/ou des ectoparasites adultes.

13. Utilisation ou procédé selon une quelconque des revendications précédentes, dans laquelle ou lequel ladite composition est formulée pour être utilisée en application topique directe sous une forme sélectionnée dans le groupe constitué par un bain, un spray, un aérosol, un shampooing, une mousse, une émulsion, une solution, une crème, une poudre, une lotion, des mousses, des microémulsions et des gels.

14. Utilisation ou procédé selon la revendication 1 ou la revendication 3, dans laquelle ou lequel ledit oeuf d'ectoparasite est un oeuf de pou de tête humain et la composition est formulée pour être utilisée en application topique sur le cuir chevelu humain sous une forme sélectionnée dans le groupe constitué par un bain, un spray, un aérosol, un shampooing, une mousse, une émulsion, une solution, une crème, une poudre, une lotion, des mousses, des microémulsions et des gels.

15. Utilisation ou procédé selon une quelconque des revendications précédentes, dans laquelle ou lequel ladite composition comprend de l'eau, des solutions salines, des alcools, des glycols polyéthyléniques, de la gélatine, du lactose, du stéarate de magnésium ou de l'acide silicique.

16. Au moins un agent chélateur métallique ou un sel de celui-ci acceptable sur le plan pharmaceutique, vétérinaire ou agricole destiné à empêcher l'éclosion d'un oeuf d'ectoparasite sur un hôte humain ou animal, dans laquelle ledit au moins un agent chélateur métallique est un composé de formule (la) : 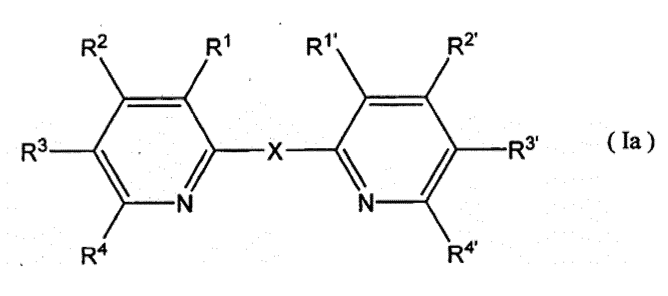
dans laquelle X est une liaison covalente CH₂-Z-CH₂ ou -Z-
dans laquelle R¹ et R^{1'} sont sélectionnés indépendamment parmi hydrogène ou C₁₋₃ alkyle
dans laquelle au moins un des R², R^{2'}, R³, R^{3'}, R⁴ et R^{4'} est un groupe méthyle et les R², R^{2'}, R³, R^{3'}, R⁴ et R^{4'} restants sont sélectionnés indépendamment parmi hydrogène ou C₁₋₃ alkyle ; et
Z est sélectionné parmi une liaison covalente -NH-, -O-, -S-, -C(O)- et -C(S)-.

17. Procédé pour empêcher l'éclosion d'un oeuf d'ectoparasite, excluant l'utilisation sur un hôte humain ou animal, consistant à exposer l'oeuf d'ectoparasite à au moins un agent chélateur métallique ou à un sel de celui-ci acceptable sur le plan pharmaceutique, vétérinaire ou agricole, dans lequel ledit au moins un agent chélateur métallique est un composé de formule (la) : 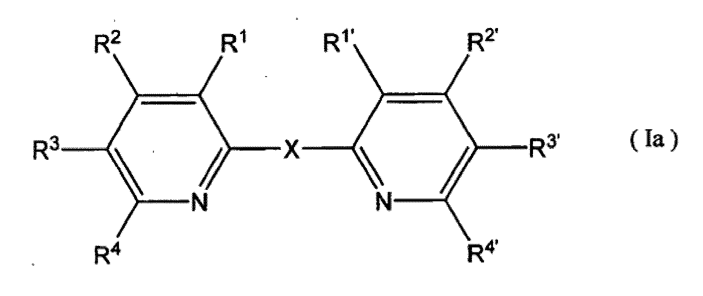
dans laquelle X est une liaison covalente CH₂-Z-CH₂ ou -Z-
dans laquelle R¹ et R^{1'} sont sélectionnés indépendamment parmi hydrogène ou C₁₋₃ alkyle
dans laquelle au moins un des R², R^{2'}, R³, R^{3'}, R⁴ et R^{4'} est un groupe méthyle et les R², R^{2'}, R³, R^{3'}, R⁴ et R^{4'} restants sont sélectionnés indépendamment parmi hydrogène ou C₁₋₃ alkyle ; et
Z est sélectionné parmi une liaison covalente -NH-, -O-, -S-, -C(O)- et -C(S)-.

18. Procédé selon la revendication 17, dans laquelle l'oeuf d'ectoparasite est présent sur des plantes hôtes comprenant : cultures céréalières, arbres fruitiers, coton, cultures de graines oléagineuses, plantes ornementales, fleurs, vignes, cultures racines, plantes fourragères et légumes, ou sur des sites de reproductions tels que maisons et bâtiments, enclos pour animaux domestiques et d'élevage, moquettes, couvertures, rideaux et meubles.

19. Utilisation, procédé ou agent selon une quelconque des revendications précédentes, dans laquelle ou lequel le composé de la formule (la) est du 5,5'-diméthyle-2,2'-dipyridyle ou un sel de celui-ci acceptable sur le plan pharmaceutique, vétérinaire ou agricole.
